# EUROPÄISCHE PATENTSCHRIFT

(11) **EP 3 233 257 B1**
(45) Veröffentlichungstag und Bekanntmachung des Hinweises auf die Patenterteilung: **14.04.2021**
(21) Anmeldenummer: 15795160.9
(22) Anmeldetag: 17.11.2015
(51) Int. Cl.: B01F 13/00, B65D 81/32, B65D 83/00, B01F 5/00, B01F 15/02, B01F 15/00, A61Q 5/08, A61Q 5/10, A61K 8/22, A61K 8/34

(54) **ZWEI-KOMPONENTEN-PRODUKTE IN BEUTELN ZUR OXIDATIVEN FARBVERÄNDERUNG VON KERATINISCHEN FASERN**
TWO-COMPONENT PRODUCTS IN BAGS FOR THE OXIDATIVE DYEING OF KERATIN FIBRES
PRODUIT À DEUX COMPOSANTS EN SACHETS POUR LA COLORATION OXYDATIVE DE FIBRES KÉRATINIQUES

(30) Priorität: 18.12.2014 DE 102014226366
(43) Veröffentlichungstag der Anmeldung: 25.10.2017
(73) Patentinhaber: Henkel AG & Co. KGaA, 40589 Düsseldorf (DE)
(72) Erfinder: MÜLLER, Burkhard, 40221 Düsseldorf (DE)
(86) Internationale Anmeldenummer: PCT/EP2015/076763
(87) Internationale Veröffentlichungsnummer: WO 2016/096280

(56) Entgegenhaltungen:
- WO-A1-2015/028016
- WO-A1-2015/028017
- WO-A1-2015/028018
- DE-A1-102008 017 104
- US-A1- 2009 236 363
- US-A1- 2011 215 113

## Beschreibung

Die vorliegende Erfindung liegt auf dem Gebiet der Kosmetik und betrifft Produkte zur oxidativen Farbveränderung von keratinischen Fasern, welche mindestens zwei getrennt voneinander konfektionierte Zubereitungen (A) und (B) umfassen. Die Zubereitung (A) enthält Wasserstoffperoxid und spezielle Komplexbildner, und die Zubereitung (B) enthält mindestens ein Alkalisierungsmittel. Weiterhin sind die beiden Zubereitungen (A) und (B) getrennt voneinander in zwei Behältnissen (A) und (B) konfektioniert, die bevorzugt Beutel sind und die jeweils aus mindestens zwei Schichten bestehen. Bei der inneren Schicht jedes Behältnisses handelt es sich um eine synthetische Polymerschicht, während die äußere Schicht jedes Behältnisses jeweils eine Metallschicht, die bevorzugt eine Aluminiumfolie ist, umfasst.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten.

Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln - in der Regel Wasserstoffperoxid - untereinander die eigentlichen Farbstoffe ausbilden. Oxidationsfärbemittel zeichnen sich durch hervorragende, lang anhaltende Färbeergebnisse aus.

Oxidative Farbveränderungsmittel kommen üblicherweise in Form von Zweikomponenten-Mitteln auf den Markt, bei welchen zwei verschiedene Zubereitungen in zwei getrennten Containern separat konfektioniert vorliegen und kurz vor der Anwendung miteinander vermischt werden müssen.

Bei der ersten Zubereitung handelt es sich um eine - aus Stabilitätsgründen in der Regel sauer eingestellte - Formulierung, welche als Oxidationsmittel Wasserstoffperoxid in Konzentrationen von 1 bis 12 Gew.-% enthält. Die Oxidationsmittelformulierung liegt meist in Form einer Emulsion oder Dispersion vor und wird in der Regel in einer Kunstoff-Flasche mit wiederverschließbarer Auslassöffnung zur Verfügung gestellt (Entwicklerflasche).

Bei der zweiten Zubereitung handelt es sich um eine alkalisch eingestellte Formulierung, die oft in Form einer Creme oder eines Gels vorliegt und welche, sofern gleichzeitig mit der Aufhellung auch eine Farbänderung gewünscht wird, zusätzlich auch die Oxidationsfarbstoffvorprodukte enthält. Diese zweite Komponente wird in den meisten Fällen in einer Tube, seltener in einem Kunststoffcontainer oder in einer Glasflasche bereitgestellt.

Zur Herstellung der anwendungsbereiten Mischung muss der Verbraucher beide Zubereitungen kurz vor der Anwendung miteinander vermischen. Hierfür wird normalerweise die alkalische Creme- oder Gelkomponente aus der Tube bzw. dem Glas- oder Kunststoffcontainer komplett in die Entwicklerflasche überführt, dann werden beide Komponenten durch Schütteln möglichst vollständig und homogen miteinander vermischt und schließlich über eine Auslassöffnung im Kopf der Entwicklerflasche entnommen.

Dieser separate Mischungsvorgang hat für den Anwender jedoch eine Reihe von Nachteilen. So kann durch die unvollständige Entleerung der Tube das Mengenverhältnis beider Komponenten verändert werden, was zu Abweichungen im gewünschten Farbergebnis führt. Bei zu kurzem Verschütteln bzw. Vermischen beider Komponenten ist die Anwendungsmischung inhomogen, die Folge hiervon ist ein ungleichmäßiges Farbergebnis. Darüber hinaus ist es auch aus Gründen des Anwenderkomforts wünschenswert, auf diesen Mischungsschritt komplett zu verzichten.

Zur Vermeidung dieser Nachteile wurden Mehrkammerbehältnisse mit gemeinsamer Austrittsöffnung entwickelt, bei denen die beiden Komponenten im Ventil oder Spender während des Austritts gemischt werden. Die Entnahme der Anwendungsmischung über den Spender macht ein Vermischen der Komponenten durch den Verbraucher überflüssig und hat den Anwendungskomfort deutlich erhöht.

In einer für den Anwender besonders komfortablen Ausführungsform handelt es sich bei den Mehrkammerbehältnissen mit gemeinsamer Austrittsöffnung um ein Aerosol-Produkt. Bedingt durch das im Aerosolprodukt anwesende Treibmittel können die beiden Zubereitungen per Druck auf das Ventil oder den Spender gleichmäßig in Form eines homogenen Schaums vom Anwender entnommen werden. Da die beiden Zubereitungen während der Entnahme im Ventil oder Spender vermischt werden, ist ein mit Aufwand verbundenes Vermischen, Verschütteln oder Verrühren durch den Anwender nicht mehr notwendig.

DE 102008017104 A1 offenbart einen Spender zur Bevorratung und Abgabe eines mehrkomponentigen Haarbehandlungsmittels. Diese Spender besitzen eine Pump-Vorrichtung und werden als kartuschenartige Vorratsbehälter beschrieben, die nach dem Prinzip von Kartuschenspritzpistolen konstruiert sind.

US 2009/0236363 A1 beschreibt ein "Bag-on-Valve-System" in Form eines oxidationsmittelhaltigen Spray-Reinigungsmittels, welches die Konfektionierung einer "all-in-one" Zubereitung ermöglicht.

In US 2011/0215113 A1 wird ein "Bag-on-Valve-System" beschrieben, welches die gleichzeitige Anwendung von mehreren Zubereitungen zulässt. Bei den in US 2011/0215113 A1 offenbarten Zubereitungen handelt es sich um Reinigungsmittel, die auch ein Oxidationsmittel als Bleichaktivator enthalten können.

WO 2015/028016 A1, WO 2015/028017 A1 und WO 2015/028018 A1 beschreiben jeweils ein Produkt zur Färbung bzw. Aufhellung von Haaren, das einen Spender mit zwei getrennten Kammern (A) und (B) umfasst, wobei sich in diesen beiden Kammern die Zubereitungen (a) und (b) befinden.

Die Außenhülle sehr vieler Aerosol-Behältnisse besteht aus Metall. Die beiden zu Beginn des oxidativen Farbänderungsprozesses zu vermischenden Zubereitungen enthalten mit starken Alkalisierungsmitteln und Oxidationsmitteln sehr reaktive Chemikalien, die an die Lagerung und Konfektionierung im Aerosol-System spezielle Anforderungen stellen. Zur Vermeidung von unerwünschten Nebenreaktionen (wie z. B. der Korrosion des metallenen Aerosol-Behältnisses) werden diese beiden Zubereitungen daher nicht direkt in zwei Kammern eines Aerosol-Behälters abgefüllt, sondern werden zunächst in zwei getrennte Beutel gefüllt, die sich dann beide im Inneren des metallenen Aerosol-Behältnisses befinden.

Zur Entnahme der beiden Zubereitungen aus den Beuteln ragen im Regelfall zwei mit dem Ventil verbundene Röhren in jeden Beutel. Bei Betätigung des Ventils werden dann beide Zubereitungen aus jedem Beutel durch das Treibgas über die beiden Röhren in Richtung Ventil gepresst, vermischen sich im, kurz unterhalb oder kurz oberhalb des Ventils miteinander und treten dann Form der Anwendungsmischung aus dem Ventil aus.

Die genaue Konstruktion dieser mit Beuteln versehenen Zwei-Kammer-Aerosol-Systeme wird beispielsweise in EP 2 738 117 A1 oder in US 2009/0108021 A1 offenbart, auf die an dieser Stelle vollinhaltlich Bezug genommen wird.

Um ein Entweichen der Inhaltsstoffe (insbesondere der reaktiven Agenzien) aus den Beuteln zu verhindern, bestehen die Beutel in der Regel aus mindestens zwei Schichten, einer inneren Polymerschicht und eine äußeren Metallschicht (wie beispielsweise Aluminium). Die äußere Metall- bzw. Aluminiumschicht besitzt eine hohe Dichtigkeit gegenüber Gasen und verhindert das Entweichen von Sauerstoff (der durch den Abbau von Wasserstoffperoxid entsteht), das Verdunsten von Wasser oder Ammoniak und auch das Entweichen aller weiteren flüchtigen Bestandteile wie beispielsweise Lösungsmittel.

Um einen direkten Kontakt der Zubereitungen mit der Metall bzw. Aluminiumfolie zu verhindern, wird auf die Aluminiumschichten auf der Innenseite der Beutel mindestens eine zusätzliche Polymerschicht aufgetragen. Bei dieser inneren Polymerschicht handelt es sich beispielsweise um eine Schicht aus einem synthetischen Polyolefin-Polymer. Diese beiden Schichten können zusätzlich noch durch eine Klebeschicht, die aus einem Adhäsionspolymer bestehen kann, miteinander verbunden sein. In diesem Fall umfassen die Beutel demnach mindestens drei Schichten: eine innere Schicht aus einem synthetischen Polyolefin-Polymer, eine mittlere Schicht aus einem Adhäsions-Polymer und eine äußere Schicht aus einer Aluminiumfolie.

Zur weiteren Verkleidung oder zum Schutz der äußeren Metall bzw. Aluminiumschicht kann diese auf der Außenseite auch nochmals zusätzlich mit einer weiteren Polymerschicht beschichtet oder behaftet sein. In diesem Fall umfassen die Beutel mindestens vier Schichten: eine innere Schicht aus einem synthetischen Polyolefin-Polymer, eine mittlere Schicht aus einem Adhäsions-Polymer, eine äußere Schicht aus einer Aluminiumfolie und ganz außen nochmals eine Schicht aus einem synthetischen Polyolefin-Polymer.

Ein genereller Nachteil dieses Beutel-Aufbaus besteht nun darin, dass auch die beiden Schichten aus innerem (Polyolefin)-Polymer und gegebenenfalls mittlerem Adhäsions-Polymer das Vordringen des Wasserstoffperoxids bis zur Aluminium-Folie nicht vollständig verhindern können. Ist das Wasserstoffperoxid dann in unerwünschter Weise durch beide Polymerschichten hindurch diffundiert, so können Wasserstoffperoxid und Aluminium miteinander reagieren, was zum Abbau des Wasserstoffperoxids, zur Entstehung von Sauerstoff und zum Aufblähen des Beutels führen kann. Aufgrund dieser Nebenreaktionen ist die Lagerstabilität der Wasserstoffperoxid enthaltenden Zubereitungen in den zuvor beschriebenen Beuteln stark reduziert.

Es war daher die Aufgabe der vorliegenden Erfindung, ein neues Produkt zur oxidativen Farbveränderung aufzufinden, welches auf einem mit Beuteln versehenen Mehr-Kammer-Aerosol-System basiert und welches auch die langfristige Lagerung einer Wasserstoffperoxid enthaltenden Zubereitung in diesen Beuteln ermöglicht.

Überraschenderweise konnte nun gefunden werden, dass der Zusatz spezieller Komplexierungsmittel bzw. Chelatbildner zu der Zubereitung, die das Wasserstoffperoxid enthält, die Reaktion des Wasserstoffperoxids mit der Metallschicht der Beutel in einem Mehr-Kammer-Aerosol-System verhindert.

Ein erster Gegenstand der vorliegenden Erfindung ist ein Produkt zur oxidativen Farbveränderung keratinischer Fasern, umfassend mindestens zwei getrennt voneinander konfektionierte Zubereitungen (A) und (B), wobei
- die erste Zubereitung (A) in einem kosmetischen Träger
   (A1) Wasserstoffperoxid und
   (A2) mindestens einen Chelatbildner aus der Gruppe aus 1-Hydroxyethan-1,1-diphosphonsäure (HEDP), Ethylendiamintetramethylenphosphonsäure (EDTMP), Diethylentriaminpentamethylenphosphonsäure (DTPMP), Aminotrimethylenphosphonsäure (ATMP), N,N-Bis[2-[bis(carboxymethyl)amino]ethyl]glycin, Ethylendiamin-N,N'-dibernsteinsäure (EDDS), 2-Hydroxypropylendiamin-N,N'-dibernsteinsäure (HPDDS), Ethylendiamin-N,N'-diglutarsäure (EDDG), Ethylendiamin-N,N'-bis-(orthohydroxyphenyl)essigsäure (EDDHA) und/oder einem physiologisch verträglichen Salz hiervon enthält,
- die zweite Zubereitung (B) in einem kosmetischen Träger
   (B1) mindestens ein Alkalisierungsmittel enthält,
- die Zubereitung (A) in einem ersten Behältnis (Behältnis A) konfektioniert ist, das mindestens zwei Schichten umfasst, wobei
   - eine innere Schicht des Behältnisses A eine Schicht aus einem synthetischen Polymer (I) aus der Gruppe der Polyolefine umfasst und
   - eine äußere Schicht des Behältnisses A eine Schicht aus Metall umfasst, und
- die Zubereitung (B) in einem zweiten Behältnis (Behältnis B) konfektioniert ist, das mindestens zwei Schichten umfasst, wobei
   - eine innere Schicht des Behältnisses B eine Schicht aus einem synthetischen Polymer (I) aus der Gruppe der Polyolefine umfasst und
   - eine äußere Schicht des Behältnisses B eine Schicht aus Metall umfasst,
- das erste Behältnis (Behältnis A) und das zweite Behältnis (Behältnis B) sich zusammen im Inneren eines Aerosol-Druckbehälters (C) befinden,
- der Aerosol-Druckbehälter (C) eine Auslassöffnung (D) aufweist, die mit dem Behältnis (A) und mit dem Behältnis (B) in Verbindung steht und
- der Raum zwischen den Außenwandungen der beiden Behältnisse (A) und (B) und der Innenwandung des Aerosol-Druckbehälters (C) mit mindestens einem Treibgas befüllt ist.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Die in den Behältnissen (A) und (B) befindlichen Zubereitungen (A) und (B) enthalten die wesentlichen Inhaltsstoffe jeweils in einem kosmetischen Träger, bevorzugt in einem geeigneten wässrigen, alkoholischen oder wässrig-alkoholischen Träger. Zum Zwecke der oxidativen Farbänderung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei den Zubereitungen (A) und/oder (B) um Cremes oder Emulsionen.

Unter dem erfindungsgemäß verwendeten Begriff "Mittel zur oxidativen Farbveränderung" werden oxidative Färbemittel verstanden, welche die Färbung der Keratinfasern oxidativ, d.h. durch den Einsatz des in Zubereitung (A) enthaltenen Wasserstoffperoxids, verändern.

Enthält das erfindungsgemäße Produkt neben dem Wasserstoffperoxid keine weiteren Farbstoffe, so handelt es sich bei der oxidativen Farbänderung ausschließlich um eine Blondierung, Bleiche oder Aufhellung, die durch die Zerstörung der dem Keratin eigenen Farbpigmente, dem Melanin, hervorgerufen wird. Zusätzlich können die erfindungsgemäßen Produkte zur oxidativen Farbänderung jedoch auch noch eine oder mehrere Oxidationsfarbstoffvorprodukte (vom Entwickler- und vom Kupplertyp) enthalten. Das im Produkt enthaltene Wasserstoffperoxid initiiert in diesem Fall eine Farbstoffbildungsreaktion zwischen Entwickler und Kuppler, und die oxidative Farbänderung ist in diesem Fall sowohl eine - mehr oder weniger starke Aufhellung - als auch eine Färbung.

Zuätzlich enthaltende Oxidationsfarbstoffvorprodukte werden üblicherweise zusammen mit dem Alkalisierungsmittel in der Zubereitung (B) konfektioniert.

Das erfindungsgemäße Produkt umfasst mindestens zwei getrennt voneinander konfektionierte Zubereitungen (A) und (B).

Kennzeichnend für die Zubereitung (A) ist ihr Gehalt an
(A1) Wasserstoffperoxid und
(A2) mindestens einem Chelatbildner aus der Gruppe aus 1-Hydroxyethan-1,1-diphosphonsäure (HEDP), Ethylendiamintetramethylenphosphonsäure (EDTMP), Diethylentriaminpentamethylenphosphonsäure (DTPMP), Aminotrimethylenphosphonsäure (ATMP), N,N-Bis[2-[bis(carboxymethyl) amino]ethyl]glycin, Ethylendiamin-N,N'-dibemsteinsäure (EDDS), 2-Hydroxypropylendiamin-N,N'-dibernsteinsäure (HPDDS), Ethylendiamin-N,N'-diglutarsäure (EDDG), 2-Hydroxypropylendiamin-N,N'-dibernsteinsäure (HPDDS), Ethylendiamin-N,N'-bis-(orthohydroxyphenyl)essigsäure (EDDHA), Diethylentriaminpentaessigsäure (DTPA) und/oder einem physiologisch verträglichen Salz hiervon.

In der Zubereitung (A) wird Wasserstoffperoxid (A1) selbst oder eines seiner festen Anlagerungsprodukte an organische und anorganische Verbindungen eingesetzt. Als feste Anlagerungsprodukte kommen erfindungsgemäß insbesondere die Anlagerungsprodukte an Harnstoff, Melamin, Polyvinylpyrrolidinon, Natriumcarbonat sowie Natriumborat in Frage.

Bevorzugt wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte Zubereitungen (A) sind dadurch gekennzeichnet, dass sie, bezogen auf das Gesamtgewicht der Zubereitung (A), Wasserstoffperoxid (A1) in einer Menge von 0,5 bis 20,0 Gew.-%, bevorzugt 1,5 bis 17,0 Gew.-%, weiter bevorzugt von 1,5 bis 15,0 Gew.-% und besonders bevorzugt von 2,5 bis 12,0 Gew.-% enthalten.

In einer besonders bevorzugten Ausführungsform ist ein erfindungsgemäßes Produkt dadurch gekennzeichnet, dass die erste Zubereitung (A) - bezogen auf das Gesamtgewicht der Zubereitung (A)-(A1) Wasserstoffperoxid in einer Menge von 0,5 bis 20,0 Gew.-%, bevorzugt 1,5 bis 17,0 Gew.-%, weiter bevorzugt von 1,5 bis 15,0 Gew.-% und besonders bevorzugt von 2,5 bis 12,0 Gew.-% enthält.

Ein wesentliches und ganz zentrales Merkmal des erfindungsgmäßen Produktes ist der Zusatz mindestens eines Chelatbildners aus der Gruppe (A2) zu der Zubereitung (A), welche auch das Wasserstoffperoxid (A1) enthält.

Chelatbildner können auch als Komplexbildner bezeichnet werden und sind Verbindungen, die zur Ausbildung von Chelaten befähigt sind. Der Begriff Chelat ist eine Sammelbezeichnung für cyklische Verbindungen, bei denen Metalle und Gruppierungen mit einsamen Elektronenpaaren an einer Ringbildung beteiligt sind. Diese Ringbildung erfolgt hierbei durch Ausbildung von koordinativen Bindungen des zentralen Metallions mit einem oder mehreren mehrzähnigen Liganden, d.h. Liganden, die mehr als ein freies Elektronenpaar besitzen.

Der bzw. die in der Zubereitung (A) enthaltenen Chelatbildner (A2) sind ausgewählt aus der Gruppe aus
- 1-Hydroxyethan-1,1-diphosphonsäure (HEDP),
- Ethylendiamintetramethylenphosphonsäure (EDTMP),
- Diethylentriaminpentamethylenphosphonsäure (DTPMP),
- Aminotrimethylenphosphonsäure (ATMP),
- N,N-Bis[2-[bis(carboxymethyl)amino]ethyl]glycin
- Ethylendiamin-N,N'-dibemsteinsäure (EDDS)
- 2-Hydroxypropylendiamin-N,N'-dibernsteinsäure (HPDDS)
- Ethylendiamin-N,N'-diglutarsäure (EDDG)
- Ethylendiamin-N,N'-bis-(orthohydroxyphenyl)essigsäure (EDDHA) und/oder einem physiologisch verträglichen Salz hiervon.

1-Hydroxyethan-1,1-diphosphonsäure (HEDP) wird alternativ auch als Etidronsäure bezeichnet, ist eine Verbindung mit der Formel (I) und besitzt die CAS-Nummer 2809-21-4.

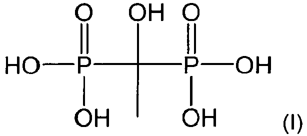

Geeignete physiologisch verträgliche Salze von HEDP sind beispielsweise das Mono-, Di-, Tri- oder das Tetranatriumsalz, oder das Mono-, Di-, Tri- oder Tetrakaliumsalz.

Bei Ethylendiamintetramethylenphosphonsäure (EDTMP) handelt es sich um eine Verbindung der Formel (II), die Substanz besitzt die CAS-Nr 1429-50-1.

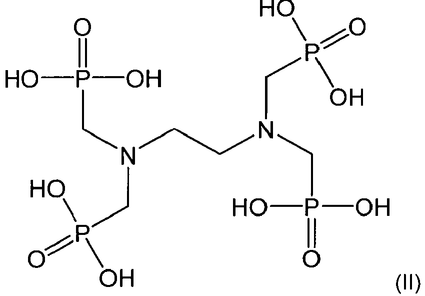

Geeignete physiologisch verträgliche Salze von EDTMP sind beispielsweise das Mono-, Di-, Tri- oder das Tetranatriumsalz, oder das Mono-, Di-, Tri-oder Tetrakaliumsalz.

Bei Diethylentriaminpentamethylenphosphonsäure (DTPMP) handelt es sich um eine Verbindung der Formel (III), die Substanz hat die CAS-Nr. 15827-60-8.

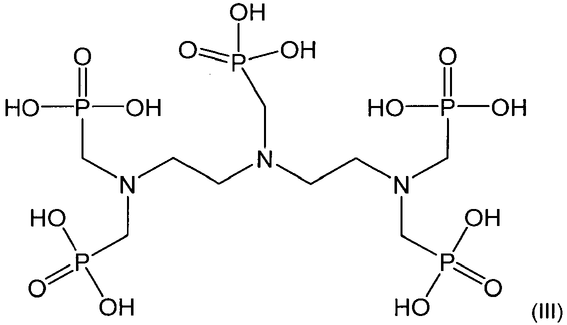

Geeignete physiologisch verträgliche Salze von DTPMP sind das Mono-, Di, Tri-, Tetra- und Pentanatriumsalz dieser Verbindung, Mono-, Di, Tri-, Tetra- und Pentakaliumsalz dieser Verbindung. Aminotrimethylenphosphonsäure (ATMP) wird alternativ auch als Nitriolotris(methylenphosphon-säure) bezeichnet. ATMP besitzt die Formel (IV) und hat die CAS-Nr.6419-19-8.

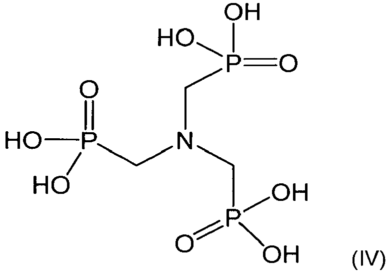

Physiologisch verträgliche Salze hiervon sind beispielsweise das Mono-, Di- oder Trinatriumsalz von ATMP, oder das Mono-, Di- oder Trikaliumsalz dieser Verbindung.

Bei N,N-Bis[2-[bis(carboxymethyl)amino]ethyl]glycin handelt es sich um eine Verbindung der Formel (V). Alternativnamen für diese Verbindung sind Diethylentriamin-pentaessigsäure (DTPA), oder auch 1,1,4,7,7-Diethylenetriaminepentaessigsäure. Die Verbindung besitzt die CAS-Nr. 67-43-6.

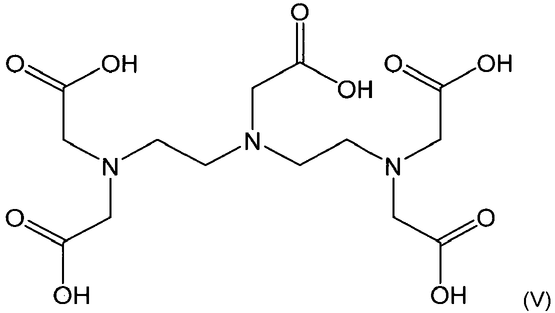

Geeignete physiologisch verträgliche Salze hiervon sind beispielsweise das Mono-, Di-, Tri-, Tetra- oder Pentanatriumsalz oder das Mono-, Di-, Tri-, Tetra- oder Pentakaliumsalz dieser Verbindung. Bei Bei Ethylendiamin-N,N'-dibemsteinsäure (EDDS), alternativ auch als Ethylendiamindisuccinat bezeichnet, handelt es sich um eine Verbindung der Formel (VI). Die Verbindung besitzt die CAS-Nr. 20846-91-7.

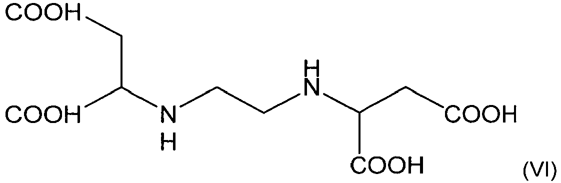

Ein geeignetes physiologisch verträgliches Salz ist beispielsweise das Mono-, Di-, Tri- oder Tetranatriumsalz der Verbindung, oder das Mono-, Di-, Tri- oder Tetrakaliumsalz dieser Verbindung.

Bei 2-Hydroxypropylendiamin-N,N'-dibernsteinsäure (HPDDS) handelt es sich um eine Verbindung der Formel (VII).

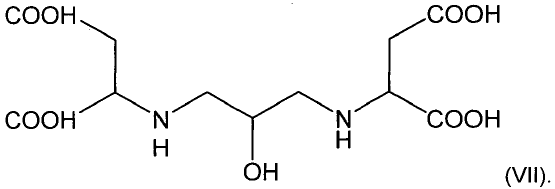

Ein geeignetes physiologisch verträgliches Salz ist beispielsweise das Mono-, Di-, Tri- oder Tetranatriumsalz der Verbindung, oder das Mono-, Di-, Tri- oder Tetrakaliumsalz dieser Verbindung.

Bei Ethylendiamin-N,N'-diglutarsäure (EDDG) handelt es sich um eine Verbindung der Formel (VIII).

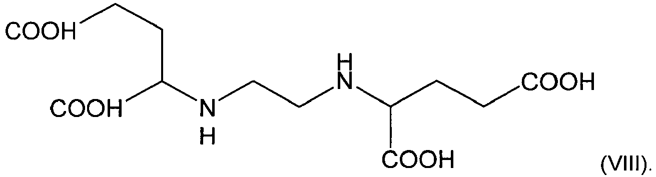

Ein geeignetes physiologisch verträgliches Salz ist beispielsweise das Mono-, Di-, Tri- oder Tetranatriumsalz der Verbindung, oder das Mono-, Di-, Tri- oder Tetrakaliumsalz dieser Verbindung. Ethylendiamin-N,N'-bis-(orthohydroxyphenyl)essigsäure (EDDHA) ist eine Verbindung der Formel (IX) und besitzt die CAS-Nr. 1170-02-1.

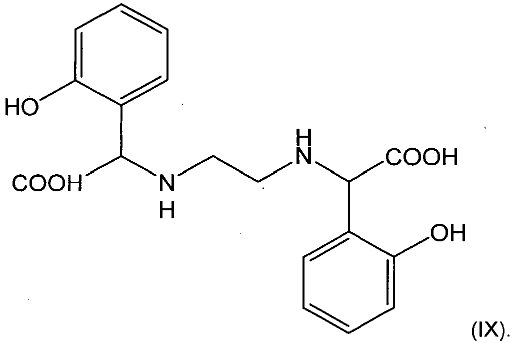

Im Zuge der zu dieser Erfindung führenden Arbeiten hat sich herausgestellt, dass der Zusatz eines oder mehrerer Chelatbildner der Formeln (I) bis (IX) zur Zubereitung (A) gewährleistet, dass die Zubereitung (A), welche das Wasserstoffperoxid (A1) enthält, in einem mehrschichtigen Container bzw. Beutel mit einer äußeren Metall- oder Aluminium-Außenfolie gelagert werden kann, ohne dass es zu unerwünschten Reaktionen zwischen dem Wasserstoffperoxid und Aluminiumfolie kommt. Auf diese Weise wurde es möglich, die Zubereitung (A) auch für einen längeren Zeitraum von mehreren Monaten in einem entsprechenden Beutel zu lagern. Die Entstehung von Sauerstoff und das damit verbundene Aufblähen des Behältnisses bzw. des Beutels während das Lagerzeitraums konnte auf diese Weise stark reduziert bzw. sogar verhindert werden.

Zur Optimierung der Lagerstabilität werden der bzw. die Chelatbildner aus der Gruppe (A2) bevorzugt in bestimmten Mengenbereichen in der Zubereitung (A) eingesetzt. Eine Erhöhung der Lagerstabilität konnte bereits bei geringen Einsatzmengen an Chelatbildner (A2) beobachtet werden.

Jedoch konnte die Reaktion des Wasserstoffperoxids mit der Metallfolie des Beutels insbesondere dann effektiv verhindert werden, wenn der bwz. die Chelatbildner in einem bestimmten Mengenbereich zu der Zubereitung (A) hinzugefügt wurden. So werden die Chelatbildner (A2) in der Zubereitung (A) - bezogen auf das Gesamtgewicht der Zubereitung (A) - insbesondere im Bereich von 0,01 bis 5,5 Gew.-%, bevorzugt von 0,1 bis 4,5 Gew.-%, weiter bevorzugt von 0,3 bis 3,5 Gew.-% und besonders bevorzugt von 0,7 bis 2,5 Gew.-% eingesetzt. Berechnungsgrundlage für diese Mengenangaben in Gew.-% ist hierbei das Gesamtgewicht aller Chelatbildner aus der Gruppe (A2), das zum Gesamtgewicht der Zubereitung (A) in Relation gesetzt wird.

In einer weiteren ganz besonders bevorzugten Ausführungsform ist ein erfindungsgemäßes Produkt dadurch gekennzeichnet, dass die erste Zubereitung (A) - bezogen auf das Gesamtgewicht der Zubereitung (A) - einen oder mehrere Chelatbildner (A2) in einer Gesamtmenge von 0,01 bis 5,5 Gew.-%, bevorzugt von 0,1 bis 4,5 Gew.-%, weiter bevorzugt von 0,3 bis 3,5 Gew.-% und besonders bevorzugt von 0,7 bis 2,5 Gew.-% enthält.

Zur Erhöhung der Lagerstabitlität der Zubereitung (A) im erfindungsgemäßen Behältnis (Behältnis A) haben sich alle Chelatbildner der Formeln (I) bis (IX) als geeignet erwiesen. In diesem Zusammenhang hat sich jedoch herausgestellt, dass insbesondere die Chelatbildner, die mindestens eine Phosphonsäure-Gruppe besitzen, ganz besonders gut geeignet sind. Besonders effektiv bei der Erhöhung der Lagerstabilität sind daher die Chelatbildner aus der Gruppe
- 1-Hydroxyethan-1,1-diphosphonsäure (HEDP),
- Ethylendiamintetramethylenphosphonsäure (EDTMP),
- Diethylentriaminpentamethylenphosphonsäure (DTPMP),
- Aminotrimethylenphosphonsäure (ATMP),
und/oder die physiologisch verträglichen Salze hiervon.

In einer weiteren ganz besonders bevorzugten Ausführungsform ist ein erfindungsgemäßes Produkt dadurch gekennzeichnet, dass die erste Zubereitung (A) -
(A2) mindestens einen Chelatbildner aus der Gruppe aus 1-Hydroxyethan-1,1-diphosphonsäure (HEDP), Ethylendiamintetramethylenphosphonsäure (EDTMP), Diethylentriaminpentamethylenphosphonsäure (DTPMP), Aminotrimethylenphosphonsäure (ATMP), und/oder einem physiologisch verträglichen Salz hiervon enthält.

Innerhalb dieser Gruppe wiederum sind der Chelatbildner 1-Hydroxyethan-1,1-diphosphonsäure (HEDP) und die physiologisch verträglichen Salze hiervon ganz besonders bevorzugt, da mit diesem Chelatbildner die besten Ergebnisse erzielt werden konnten.

In einer weiteren ganz besonders bevorzugten Ausführungsform ist ein erfindungsgemäßes Produkt dadurch gekennzeichnet, dass die erste Zubereitung (A) -
(A2) als Chelatbildner 1-Hydroxyethan-1,1-diphosphonsäure (HEDP) und/oder ein physiologisch verträgliches Salz hiervon enthält.

Weiterhin auch ganz besonders bevorzugt ist ein erfindungsgemäßes Produkt, das dadurch gekennzeichnet ist, dass die erste Zubereitung (A) - bezogen auf das Gesamtgewicht der Zubereitung (A)
(A2) 1-Hydroxyethan-1,1-diphosphononsäure (HEDP) und/oder deren physiologisch verträgliche Salze in einer Gesamtmenge von 0,01 bis 5,5 Gew.-%, bevorzugt 0,1 bis 4,5 Gew.-%, weiter bevorzugt von 0,3 bis 3,5 Gew.-% und besonders bevorzugt von 0,7 bis 2,5 Gew.-% enthält.

Bei den Chelatbildnern aus der Gruppe (A2) handelt es sich um die Verbindungen der Formeln (I) bis (IX) und/oder um die phyiologisch verträglichen Salze hiervon. Physiologisch verträglich bedeutet in diesem Zusammenhang zum Einsatz im kosmetischen Mittel (d.h. zur Anwendung auf dem menschlichen Haar und der menschlichen Haut) geeignet. Besonders geeignete physiologisch verträgliche Salze sind die Natriumsalze, die Kaliumsalze und/oder die Ammoniumsalze (NH₄⁺) der Chelatbildner der Formeln (I) bis (IX).

Die Einsatzmenge an Wasserstoffperoxid in der Zubereitung (A) wird bestimmt durch den gewünschten Aufhelleffekt auf der keratinischen Faser. Wird nur eine leichte Aufhellung oder vornehmlich eine Färbung der keratinischen Fasern gewünscht, so kann ein Wasserstoffperoxid-Gehalt von 1,0 bis 2,5 Gew.-% - bezogen auf das Gesamtgewicht der Zubereitung (A) ausreichend sein. Sollen die keratinischen Fasern jedoch stärker aufgehellt oder blondiert werden, so wird auch ein höherer Gehalt an Wasserstoffperoxid von beispielsweise bis zu 17,0 Gew.-% (bezogen auf das Gesamtgewicht der Zubereitung (A)) gewählt.

In diesem Zusammenhang hat sich herausgestellt, dass die Reaktionen zwischen Wasserstoffperoxid und der äußeren Metall- bzw. Aluminiumfolie des Beutels mit Erhöhung der Wasserstoffperoxid Konzentration zunehmen. Zur Vermeidung dieser Reaktionen sollte daher bei höheren Einsatzmengen an Wasserstoffperoxid (A1) auch eine höhere Einsatzmenge an Chelatbildnern aus der Gruppe (A2) gewählt werden.

Mit anderen Worten besteht hinsichtlich der Lagerstabilität der Zubereitung (A) im Behältnis (A) ein Zusammenhang zwischen der in der Zubereitung (A) enthaltenen Menge an Wasserstoffperoxid (A1) und der Gesamtmenge der in der Zubereitung (A) enthaltenen Chelatbildner aus der Gruppe (A2). Aus diesem Grund sollte das Gewichtsverhältnis (A1)/(A2) zur möglichst vollständigen Unterdrückung aller Reaktionen zwischen Wasserstoffperoxid und der äußeren Metallschicht des Behältnisses bzw. Beutels auf den optimalen Wert eingestellt sein.

Als ganz besonders vorteilhaft hat es sich hierbei herausgestellt, wenn das das Gewichtsverhältnis aus der Gesamtmenge des in der Zubereitung (A) enthaltenen Wasserstoffperoxids (A1) zur Gesamtmenge der in der Zubereitung (A) enthaltenen Chelatbildner aus der Gruppe (A2), d.h. das Gewichtsverhältnis (A1)/(A2), bei einem Wert von 1,0 bis 30,0, bevorzugt von 2,0 bis 20,0, weiter bevorzugt von 3,0 bis 15,0 und besonders bevorzugt von 3,3 bis 13,5 liegt

Berechnungsgrundlage für alle Angaben in Gew.-% ist hierbei
- die in der Zubereitung (A) enthaltene Menge an Wasserstoffperoxid (A1), die zum Gesamtgewicht der Zubereitung (A) in Relation gesetzt wird, sowie
- die Gesamtmenge aller in der Zubereitung (A) enthaltenen Chelatbildner aus der Gruppe (A2), die zum Gesamtgewicht der Zubereitung (A) in Relation gesetzt wird.

Das Gewichtsverhältnis (A1)/(A2) wird dann durch Division dieser beiden Mengen ermittelt.

In einer weiteren ganz besonders bevorzugten Ausführungsform ist ein erfindungsgemäßes Produkt dadurch gekennzeichnet, dass das Gewichtsverhältnis aus der Gesamtmenge des in der Zubereitung (A) enthaltenen Wasserstoffperoxids (A1) zur Gesamtmenge der in der Zubereitung (A) enthaltenen Chelatbildner aus der Gruppe (A2), d.h. das Gewichtsverhältnis (A1)/(A2), bei einem Wert von 1,0 bis 30,0, bevorzugt von 2,0 bis 20,0, weiter bevorzugt von 3,0 bis 15,0 und besonders bevorzugt von 3,3 bis 13,5 liegt.

Durch Zusatz weiterer Stabilitsatoren (A3) können die Nebenreaktionen von Wasserstoffperoxid mit der metallenen äußeren Schicht des erfindungsgemäßen Behältnisses noch weiter unterdrückt werden. Als vorteilhaft hat sich in diesem Zusammenhang insbesondere der Zusatz von 2,6-Dipicolinsäure, Benzoesäure und/oder Salicylsäure erwiesen. Eine besonders gute Inhibierung von Nebenreaktionen konnte insbesondere dann beobachtet werden, wenn (A3) 2,6-Dipicolinsäure, Benzoesäure, Salicylsäure und/oder die phyiologisch verträglichen Salze dieser Verbindungen in einer Gesamtmenge von 0,05 bis 4,5 Gew.-%, bevorzugt 0,1 bis 1,0 Gew.-%, weiter bevorzugt von 0,2 bis 0,9 Gew.-% und besonders bevorzugt von 0,25 bis 0,7 Gew.-% in der Zubereitung (A) enthalten waren. Berechnungsgrundlage für alle Mengenangaben ist hierbei wieder die Gesamtmenge der vorgenannten Verbindungen (A3), die zum Gesamtgewicht der Zubereitung (A) in Relation gesetzt wird.

In einer weiteren ganz besonders bevorzugten Ausführungsform ist ein erfindungsgemäßes Produkt dadurch gekennzeichnet, dass die erste Zubereitung (A) - bezogen auf das Gesamtgewicht der Zubereitung (A) - zusätzlich
(A3) 2,6-Dipicolinsäure, Benzoesäure, Salicylsäure und/oder deren phyiologisch verträgliche Salze in einer Gesamtmenge von 0,05 bis 1,5 Gew.-%, bevorzugt 0,1 bis 1,0 Gew.-%, weiter bevorzugt von 0,2 bis 0,9 Gew.-% und besonders bevorzugt von 0,25 bis 0,7 Gew.-% enthält.

Die phyisologisch verträglichen Salze von 2,6-Dipicolinsäure, Benzoesäure, Salicylsäure sind insbesondere die Natriumsalze, Kaliumsalze und Ammoniumsalze der drei Verbindungen.

Die zweite Zubereitung (B) enthält in einem kosmetischen Träger mindestens ein Alkalisierungsmittel (B1).

Bevorzugt können das oder die Alkalisierungsmittel (B1) ausgewählt sein aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)-Alkalimetallphosphaten und (Erd-)Alkalimetallhydrogenphosphaten. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass die zweite Zubereitung (B) mindestens ein Alkalisierungsmittel (B1) enthält, welches ausgewählt ist aus der Gruppe, die gebildet wird aus Ammoniak, Alkanolaminen und basischen Aminosäuren, insbesondere aus Ammoniak, Monoethanolamin und Arginin und/oder seinen verträglichen Salzen.

Zur Erzielung optimaler Farbveränderungsergebnisse werden die Zubereitungen (A) und (B) bevorzugt auf bestimmte pH-Werte eingestellt.

Die Zubereitung (A), welche das Wasserstoffperoxid enthält, ist aus Stabilitätsgründen bevorzugt sauer eingestellt und besitzt bevorzugt einen pH-Wert im Bereich von 1,5 bis 5,0, bevorzugt von 2,0 bis 4,5, weiter bevorzugt von 2,2 bis 4,0 und besonders bevorzugt von 2,6 bis 3,5.

Die Zubereitung (B), welche das Alkalisierungsmittel (B1) enthält, ist dagegen bevorzugt auf einen alkalischen Bereich eingestellt und besitzt bevorzugt einen pH-Wert im Bereich von 7,5 bis 12,5, bevorzugt von 8,5 bis 11,5 und besonders bevorzugt von 8,9 bis 10,5.

Das anwendungsbereite Mittel zur Farbänderung, dass kurz vor der Anwendung durch Vermischen der Zubereitungen (A) und (B) hergestellt wird, ist bevorzugt ebenfalls auf einen alkalischen pH-Wert eingestellt, da durch die alkalischen pH-Werte eine ausreichende Quellung der Keratinfasern gewährleistet wird. Auch die Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels bei einem Wert von 8,0 bis 10,5, weiter bevorzugt von 8,7 bis 10,3, noch weiter bevorzugt von 9,0 bis 10,2 und besonders bevorzugt von 9,2 bis 10,1 liegen. Bei den angegebenen pH-Werten handelt es sich um Werte, die bei einer Temperatur von 22 °C mit einer Glaselektrode gemessen wurden.

In einer weiteren ganz besonders bevorzugten Ausführungsform ist ein erfindungsgemäßes Produkt dadurch gekennzeichnet, dass
- die erste Zubereitung (A) eine wässrige Zubereitung mit einem pH-Wert von 1,5 bis 5,0, bevorzugt von 2,0 bis 4,5, weiter bevorzugt von 2,2 bis 4,0 und besonders bevorzugt von 2,6 bis 3,5 ist und
- die zweite Zubereitung (B) eine wässrige Zubereitung mit einem pH-Wert von 7,5 bis 12,5, bevorzugt von 8,5 bis 11,5 und besonders bevorzugt von 8,9 bis 10,5 ist.

In der Zubereitung (B) wird der bevorzugte alkalische pH-Wert in vorteilhafter Weise durch Zugabe entsprechender Mengen des erfindungsgemäßen Alkalisierungsmittels (B1) eingestellt.

Auch wenn die Zubereitung (A) bevorzugt sauer eingestellt ist, zu kann zur genauen Einstellung des pH-Wertes doch die Zugabe geringer Mengen eines Alkalisierungsmittels notwenig werden. Erfindungsgemäß verwendbare Alkalisierungsmittel können aus der Gruppe ausgewählt werden, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)-Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.

Zur Einstellung des erfindungsgemäß bevorzugten niedrigen pH-Wertes können in der Zubereitung (A) beispielsweise entsprechende Mengen an Chelatbildner aus der zuvor beschriebenen Gruppe (A2) in ihrer jeweiligen Säureform eingesetzt werden. Zusätzlich können in den Zubereitungen (A) und/oder (B) zur Feineinstellung des gewünschten pH-Wertes jedoch auch noch eine oder mehrere weitere Säuren verwendet werden. Geeignete Säuren sind hierbei beispielsweise anorganische Säuren wie Salzsäure, Schwefelsäure und/oder Phosphorsäure. Es können jedoch ebenfalls auch organische Säuren wie beispielsweise Essigsäure, Milchsäure, Zitronensäure, Weinsäure oder Äpfelsäure eingesetzt werden. In diesem Zusammenhang bevorzugt sind insbesondere die geruchsarmen organischen Säuren wie Milchsäure, Zitronensäure, Weinsäure und/oder Äpfelsäure.

Wenn das erfindungsgemäße Produkt als reines Aufhellmittel eingesetzt werden soll, so enthält die Zubereitung (B) keine weiteren Farbstoffe. Bei dem erfindungsgemäßen Produkt kann es sich jedoch auch um ein oxidatives Färbemittel handeln. In diesem Fall werden der Zubereitung (B) noch weitere Oxidationsfarbstoffvorprodukte vom Entwickler und/oder vom Kupplertyp zugesetzt.

Bevorzugte weitere Entwicklerkomponenten werden ausgewählt aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetra-oxadecan, 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Besonders bevorzugte zusätzliche Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin und/oder 4,5-Diamino-1-(2-hydroxyethyl)-pyrazol sowie deren physiologisch verträglichen Salze.

Besonders intensive Färbungen liefern die Entwickler aus der Gruppe aus p-Phenylendiamin, p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4-Aminophenol, 4-Amino-3-methylphenol, 4,5-Diamino-1-(2-hydroxyethyl)pyrazol, 2,4,5,6-Tetraaminopyrimidin, 4-Hydroxy-2,5,6-triaminopyrimidin und/oder 2-Hydroxy-4,5,6-triaminopyrimidin. Aus diesem Grund ist der Einsatz dieser Entwickler und/oder ihrer physiologisch verträglichen Salze besonders bevorzugt.

In einer weiteren Ausführungsform ist ein erfindungsgemäßes Produkt dadurch gekennzeichnet, dass die zweite Zubereitung (B) mindestens ein oder mehrere Oxidationsfarbstoffvorprodukte aus der Gruppe enthält, welche gebildet wird aus p-Phenylendiamin, p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4-Aminophenol, 4-Amino-3-methylphenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triamino-pyrimidin und/oder deren physiologisch verträglichen Salzen.

Kupplerkomponenten bilden im Rahmen der oxidativen Färbung allein keine signifikante Färbung aus, sondern benötigen stets die Gegenwart von Entwicklerkomponenten. Kupplerkomponenten erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.

Gemische aus zwei oder mehreren Verbindungen aus einer oder mehreren dieser Klassen sind im Rahmen dieser Ausführungsform ebenso erfindungsgemäß.

Bevorzugt sind die Kuppler, die ausgewählt sind aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin und/oder 7-Hydroxyindolin und deren physiologisch verträglichen Salzen.

In einer weiteren Ausführungsform ist ein erfindungsgemäßes Produkt dadurch gekennzeichnet, dass die zweite Zubereitung (B) zusätzlich ein odere mehrere Oxidationsfarbstoffvorprodukte aus der Gruppe enthält, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin und/oder deren physiologisch verträglichen Salzen.

Die Zubereitung (B) kann die Entwickler und/oder Kuppler in Mengen von 0,001 bis 10,0 Gew.-% - bezogen auf das Gesamtgewicht der Zubereitung (B) - enthalten. Die Einsatzmengen hängen insbeondere davon ab, in welcher Nuance die keratinischen Fasern gefärbt werden sollen.

Zur weiteren Nuancierung kann die Zubereitung (B) auch noch ein oder mehrere direktziehende Farbstoffe aus der Gruppe der nichtionischen, anionischen und/oder kationsichen Farbstoffe enthalten.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Die Zubereitungen (A) und (b) können weiterhin zusätzliche Wirk-, Hilfs- und Zusatzstoffe enthalten, um die Aufhell- und/oder Färbeleistung zu verbessern und weitere gewünschte Eigenschaften der Mittel einzustellen. Beispielsweise kann eines oder mehrere der Mittel zusätzlich nichtionische Tenside, anionische Tenside, kationische Tenside, amphotere und/oder zwitterionische Tenside, nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

In dem erfindungsgemäßen Produkt liegt die erste Zubereitung (A) ein einem ersten Behältnis (Behältnis A) konfektioniert vor, und die zweite Zubereitung (B) liegt getrennt hiervon in einem zweiten Behältnis (Behältnis B) konfektioniert vor.

Kennzeichnendes Merkmal für das Behältnis (A) ist, dass es mindestens zwei Schichten umfasst, wobei
- eine innere Schicht des Behältnisses A eine Schicht aus einem synthetischen Polymer (I) aus der Gruppe der Polyolefine umfasst und
- eine äußere Schicht des Behältnisses A eine Schicht aus Metall umfasst.

Weiterhin kennzeichnendes Merkmal für das Behältnis (B) ist, dass es mindestens zwei Schichten umfasst, wobei
- eine innere Schicht des Behältnisses B eine Schicht aus einem synthetischen Polymer (I) aus der Gruppe der Polyolefine umfasst und
- eine äußere Schicht des Behältnisses B eine Schicht aus Metall umfasst.

Die Begriffe "innere Schicht" und "äußere Schicht" definieren hierbei die relative Lage dieser beiden Schichten zueinander. Bei Betrachtung der mit Inhalt befüllten Behältnisse (A) und (B) ist die "innere Schicht" dem Inhalt der Behältnisse jeweils näher als die "äußere Schicht". Die "innere Schicht" aus synthetischem Polymer (I) ist dem jeweiligen Beutelinhalt also immer näher als die "äußere Schicht" aus Metall.

Die erfindungsgemäßen Behältnisse (A) und (B) umfassen mindestens zwei Schichten, nämlich die "innere Schicht" aus synthetischem Polymer (I) aus der Gruppe der Polyolefine und die "äußere Schicht" aus Metall. Hierbei stellen die Bezeichnungen der "inneren Schicht" und der "äußeren Schicht" relative Begriffe, jedoch keine absoluten Begriffe dar. Hiermit ist gemeint, dass die "innere Schicht" ist nur dann auch zwangsläufig die "innerste Schicht" ist, wenn die Behältnisse (A) und (B) genau zwei Schichten umfassen. Analog ist die "äußere Schicht" nur dann auch zwangsläufig die "äußerste Schicht" wenn die Behältnisse (A) und (B) genau zwei Schichten umfassen.

Umfassen die Behältnisse (A) und (B) noch eine oder mehrere weitere Schichten, so können diese weiteren Schichten innerhalb der "inneren Schicht", zwischen der "inneren und der äußeren Schicht" und/oder auch außerhalb der "äußeren Schicht" lokalisiert sein.

Aus Kostengründen ist es bevorzugt, wenn die Behältnisse (A) und (B) aus dem gleichen Material sind, demzufolge sind die beiden inneren Schichten und die beiden äußeren Schichten der Behältnisse (A) und (B) bevorzugt jeweils gleich.

Bei den beiden Behältnissen (A) und (B) handelt es sich ganz besonders bevorzugt um Beutel.

Bevorzugt werden als Behältnisse (A) und (B) somit zwei gleiche Beutel eingesetzt. Die Gesamtschichtdicke dieser Beutel liegt hierbei bevorzugt bei einer Dicke von 2 bis 400 µm (Mikrometer), bevorzugt von 10 bis 250 µm (Mikrometer) und besonders bevorzugt von 50 bis 200 µm (Mikrometer).

In einer weiteren besonders bevorzugten Ausführungsform ist ein erfindungsgemäßes Produkt dadurch gekennzeichnet, dass
- es sich bei dem ersten Behältnis (Behältnis A) um einen Beutel mit einer Gesamt-Schichtdicke von 2 bis 400 µm (Mikrometer), bevorzugt von 10 bis 250 µm (Mikrometer) und besonders bevorzugt von 50 bis 200 µm (Mikrometer) handelt und/oder
- es sich bei dem zweiten Behältnis (Behältnis B) um einen Beutel mit einer Gesamt-Schichtdicke von 2 bis 400 µm (Mikrometer) bevorzugt von 10 bis 250 µm (Mikrometer) und besonders bevorzugt von 50 bis 200 µm (Mikrometer) handelt.

Unter der Gesamtschichtdicke wird die Summer der Schichtdicken aller im Beutel vorhandenen Einzelschichten verstanden.

In einer weiteren besonders bevorzugten Ausführungsform ist ein erfindungsgemäßes Produkt dadurch gekennzeichnet, dass
- es sich bei dem ersten Behältnis (Behältnis A) um einen Beutel mit einer Gesamt-Schichtdicke von 50 bis 200 µm (Mikrometer) handelt und
- es sich bei dem zweiten Behältnis (Behältnis B) um einen Beutel mit einer Gesamt-Schichtdicke von 50 bis 200 µm (Mikrometer) handelt.

Bei der inneren Schicht der Behältnisse (A) und (B), die jeweils eine Schicht aus einem synthetischen Polymer (I) umfasst, handelt es sich um eine Schicht aus einem Polyolefin-Polymer, wie beispielsweise ein "low density" Polyethylen (LDPE), ein "medium density" Polyethylen (MPDE), ein "high density" Polyethylen (HDPE), ein lineares "low density" Polyethylen (LLDPE), ein lineares "very low density" Poylethylen (LVLDPE), ein isotaktisches oder syndiotaktisches Polypropylen (PP), ein Ethylen-propelyn-Copolymer, ein 1-Polybuten Polymer, ein Ethylen/1-Buten Copoylmer, ein Propylen/1-Buten Copoylmer oder ein Ethylen/Propylen/1-Buten Copolymer.

Innerhalb dieser Gruppe ist das lineare "low density" Polyethylen (LLDPE) besonders bevorzugt. Die Dichte des lineare "low density" Polyethylens liegt bevorzugt bei 0,91 bis 0,94 g/cm³.

Ein erfindungsgemäßes Produkt ist dadurch gekennzeichnet, dass
- die innere Schicht des Behältnisses A eine Schicht aus einem synthetischen Polymer (I) aus der Gruppe der Polyolefine, bevorzugt aus Polyethylen, Polypropylen, einem Polyethylen/Polypropylen-Copolymer, Poly-1-buten, einem Polyethylen/Poly-1-buten-Copolymer, einem Polypropylen/Poly-1-buten-Copolymer und/oder einem Polyethylen/Polypropylen/Poly-1-buten-Copolymer umfasst, und
- die innere Schicht des Behältnisses B eine Schicht aus einem synthetischen Polymer (I) aus der Gruppe der Polyolefine, bevorzugt aus Polyethylen, Polypropylen, einem Polyethylen/Polypropylen-Copolymer, Poly-1-buten, einem Polyethylen/Poly-1-buten-Copolymer, einem Polypropylen/Poly-1-buten-Copolymer und/oder einem Polyethylen/Polypropylen/Poly-1-buten-Copolymer umfasst.

In einer explizit ganz besonders bevorzugten Ausführungsform ist ein erfindungsgemäßes Produkt dadurch gekennzeichnet, dass
- die innere Schicht des Behältnisses A eine Schicht aus einem synthetischen Polymer (I) aus Polyethylen (PE), umfasst, und
- die innere Schicht des Behältnisses B eine Schicht aus einem synthetischen Polymer (I) aus Polyethylen (PE) umfasst.

Bei den Behältnissen (A) und (B) besitzt die innere Schicht der Behältnisse aus synthetischem Polymer (I) bevorzugt eine Schichtdicke von 20 bis 200 µm (Mikrometer), bevorzugt von 40 bis 150 µm (Mikrometer) und besonders bevorzugt von 50 bis 80 µm (Mikrometer).

In einer weiteren besonders bevorzugten Ausführungsform ist ein erfindungsgemäßes Produkt dadurch gekennzeichnet, dass
- die innere Schicht des Behältnisses A eine Schicht aus einem synthetischen Polymer (I) mit einer Schichtdicke von 20 bis 200 µm (Mikrometer), bevorzugt von 40 bis 150 µm (Mikrometer) und besonders bevorzugt von 50 bis 80 µm (Mikrometer), umfasst, und/oder
- die innere Schicht des Behältnisses B eine Schicht aus einem synthetischen Polymer (I) mit einer Schichtdicke von 20 bis 200 µm (Mikrometer),, bevorzugt von 40 bis 150 µm (Mikrometer), und besonders bevorzugt von 50 bis 80 µm (Mikrometer) umfasst.

Die äußere Schicht der Behältnisse (A) und (B) umfasst jeweils eine Schicht aus Metall, bei diesem Metall handelt es sich ganz besonders bevorzugt um Aluminium, beispielsweise um jeweils eine Aluminiumfolie. Die Aluminiumfolie hat jeweils eine bevorzugte Schichtdicke von von 5 bis 50 µm (Mikrometer), bevorzugt von 7 bis 25 µm (Mikrometer) und besonders bevorzugt von 7 bis 12 µm (Mikrometer).

In einer weiteren besonders bevorzugten Ausführungsform ist ein erfindungsgemäßes Produkt dadurch gekennzeichnet, dass
- die äußere Schicht des Behältnisses A eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) umfasst und/oder
- die äußere Schicht des Behältnisses B eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) umfasst.

Anders formuliert ist in einer weiteren besonders bevorzugten Ausführungsform ist ein erfindungsgemäßes Produkt dadurch gekennzeichnet, dass
- die äußere Schicht des Behältnisses A eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) ist und/oder
- die äußere Schicht des Behältnisses B eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) ist.

In einer weiteren besonders bevorzugten Ausführungsform ist ein erfindungsgemäßes Produkt dadurch gekennzeichnet, dass
- die äußere Schicht des Behältnisses A eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) umfasst und
- die äußere Schicht des Behältnisses B eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) umfasst.

Die beiden Behältnisse (A) und (B) können auch zusätzlich noch eine oder mehrere Schichten aus mindestens einem weiteren Polymer (II) umfassen, das strukturell von dem synthetischen Polymer (I) verschieden ist.

In einer weiteren besonders bevorzugten Ausführungsform ist ein erfindungsgemäßes Produkt dadurch gekennzeichnet, dass
- die Behältnisse (A) und/oder (B) eine dritte Schicht aus einem synthetischen Polymer (II) umfassen, das strukturell von dem synthetischen Polymer (I) verschieden ist.

Bei dieser dritten Schicht kann es sich beispielsweise um eine Schicht aus einem verklebenden bzw. Adhäsions-Polymer handeln, die zwischen der inneren Schicht aus synthetischem Polymer (I) (bevorzugt Polyethylen) und äußerer metallenen Schicht (bevorzugt Aluminium) lokalisiert ist. Beginnend im Inneren des Behältnisses (A) bzw. (B) lautet die Abfolge der Schichten dann jeweils: innere Schicht aus synthetischem Polymer (I) (bevorzugt Polyethylen) - mittlere Schicht aus synthetischem Polymer (II) (Adhäsions-Polymer) - äußere Schicht aus Metall (bevorzugt Aluminiumfolie).

Ein geeignetes Adhäsionspolymer (d.h. ein synthetisches Polymer (II)) stellt beispielsweise ein Polyurethan-Copolymer, das durch Reaktion von einem Isocyanat mit einem Polyol hergestellt werden kann. Bevorzugt ist dieses Polyurethan-Copolymer vernetzt oder hochvernetzt. Als Isocycnate können beispielsweise 1,6-Hexamethylendiisocyanate, 2,2,4-Trimethyl-hexamethylendiisocyanat und Lysin Ester Diisocyanat, aliphatische Polyisocyanate wie hydrogeniertes Dihenylmethan Diisocyanat, Isophoron Diisocyanat oder hydrogeniertes Tolylen Diisocyanat, oder aromatische Polyisocyanate wie beipsielsweise Tolylen Diisocyanat, 4,4'-Diphenylmethan Diisocyanat, Nathalen Diisocyanat, Xylen Diisocyanat, Triphenylmethan Diisocyanat und Tris(4-phenylisocyanat)thiophosphat eingesetztwe werden.

Polyole, die in der Polymerisation eingesetzt werden können, sind beispielsweise Oxirane wie Ethylenoxid, Propylenoxid, Butylenoxid oder auch Tetrahydrofurn oder Ethyleglycol, Proyplenglycol, Triemthylolpropan oder Glycerin.

Alternativ kann es sich bei der dritten Schicht bei den Behältnissen (A) und/oder (B) auch noch um eine Schicht aus einem synthetischen Polymer aus der Gruppe der Polyester, Polyamide, Polyethylenterephthalate, Polybutylenterephthalate und der von Polymer (I) verschiedenen Polyolefine umfassen.

In einer weiteren bevorzugten Ausführungsform ist ein erfindungsgemäßes Produkt daher dadurch gekennzeichnet, dass
- die dritte Schicht des Behältnisses (A) eine Schicht aus einem synthetischen Polymer aus der Gruppe der Polyurethane, Polyester, Polyamide, Polyethylenterephthalate, Polybutylenterephthalate und der von Polymer (I) verschiedenen Polyolefine umfasst und/oder
- die dritte Schicht des Behältnisses (B) eine Schicht aus einem synthetischen Polymer aus der Gruppe der Polyurethane, Polyester, Polyamide, Polyethylenterephthalate, Polybutylenterephthalate und der von Polymer (I) verschiedenen Polyolefine umfasst.

In einer weiteren bevorzugten Ausführungsform ist ein erfindungsgemäßes Produkt daher dadurch gekennzeichnet, dass
- die Behältnisse (A) und (B) eine dritte Schicht aus einem synthetischen Polymer (II) besitzen, das strukturell von dem synthetischen Polymer (I) verschieden ist,
wobei
- die dritte Schicht des Behältnisses (A) eine Schicht aus einem synthetischen Polymer aus der Gruppe der Polyurethane, Polyester, Polyamide, Polyethylenterephthalate, Polybutylenterephthalate und der von Polymer (I) verschiedenen Polyolefine umfasst und
- die dritte Schicht des Behältnisses (B) eine Schicht aus einem synthetischen Polymer aus der Gruppe der Polyurethane, Polyester, Polyamide, Polyethylenterephthalate, Polybutylenterephthalate und der von Polymer (I) verschiedenen Polyolefine umfasst.

Betreffend alle weiteren Details zu den Polymerschichtdicken, Schichtabfolgen und Schichtmaterialien der Behältnisse (A) und (B) wird vollinhaltlich auf EP 2 738 117 A1 Bezug genommen.

Das Vorhandensein weiterer Schichten aus zusätzlichen Polymeren, die sich zwischen dem Beutelinhalt und der äußeren metallenen Schicht befinden, kann die Diffusion des Wasserstoffperoxids durch diese Polymerschichten verlangsamen, ggf. bis zu einem gewissen Grad inhibieren und somit die Reaktion zwischen Wasserstoffperoxid und der äußeren Aluminiumschicht des Beutels reduzieren. Gleichzeitigt stellt die Herstellung von vielfach mit verschiedenen Materialien beschichteten Beuteln jedoch einen hohen Kostenfaktor dar, der den Preis der Farbveränderungsprodukte über Gebühr erhöhen kann. Aus wirtschaftlichen Gründen ist es daher bevorzugt, wenn die erfindungsgemäßen Beutel (A) und (B) aus so wenig verschiedenen Schichten wie möglich bestehen. Durch Einsatz der erfindungswesentlichen Komplexbildner aus der Gruppe (A2) kann die Reaktion zwischen Wasserstoffperoxid und der Aluminiumfolie des Beutels nun unterdrückt oder minimiert werden, ohne das die Beschichtung der Innenseite des Beutels mit vielen weiteren Materialien nötig wäre. Eine weitere ganz besonders bevorzugte Ausführungsform ist daher dadurch gekennzeichnet, dass die Behältnisse (A) und (B) aus nicht mehr als 7, bevorzugt nicht mehr als 6, weiter bevorzugt nicht mehr als 5, noch weiter bevorzugt aus nicht mehr als 4 und ganz besonders bevorzugt aus nicht mehr als 3 verschiedenen Schichten bestehen.

In einer weiteren bevorzugten Ausführungsform ist ein erfindungsgemäßes Produkt daher dadurch gekennzeichnet, dass
- das Behältnis (A) aus nicht mehr als 7, bevorzugt nicht mehr als 6, weiter bevorzugt nicht mehr als 5, noch weiter bevorzugt nicht mehr als 4 und ganz besonders bevorzugt nicht mehr als 3 verschiedenen Schichten besteht und/oder
- das Behältnis (B) aus nicht mehr als 7, bevorzugt nicht mehr als 6, weiter bevorzugt nicht mehr als 5, noch weiter bevorzugt nicht mehr als 4 und ganz besonders bevorzugt nicht mehr als 3 verschiedenen Schichten besteht.

Unter verschiedenen Schichten werden im Sinne der vorliegenden Erfindung Schichten aus unterschiedlichen Materialien, d.h. Schichten aus unterschiedlichen Polymeren und Metallen, verstanden.

Bei dem erfindungsgemäßen Produkt handelt es sich um zwei beutelförmige Behältnisse (A) und (B), welche jeweils die Zubereitungen (A) und (B) beinhalten und welche sich zusammen in einem Aerosol-Druckbehälter (C) befinden. Der Aeorosol-Druckbehälter besitzt eine Auslassöffnung (D), die mit dem Behältnis (A) und dem Behältnis (B) in Verbindung steht.

Ein erfindungsgemäßes Produkt ist dadurch gekennzeichnet, dass
- das erste Behältnis (Behältnis A) und das zweite Behältnis (Behältnis B) sich zusammen im Inneren eines Aerosol-Druckbehälters (C) befinden,
- der Aerosol-Druckbehälter (C) eine Auslassöffnung (D) aufweist, die mit dem Behältnis (A) und mit dem Behältnis (B) in Verbindung steht und
- der Raum zwischen den Außenwandungen der beiden Behältnisse (A) und (B) und der Innenwandung des Aerosol-Druckbehälters (C) mit mindestens einem Treibgas befüllt ist.

Hierbei sind die Zusammensetzungen (A) und (B) in den Behältnissen (A) und (B) getrennt konfektioniert, können über die Auslassöffnung (D) jedoch in Kontakt gebracht werden. Solange die Auslassöffnung (z.B. das Ventil) nicht betätigt wird, sind die Zubereitungen (A) und (B) getrennt, und ein Kontakt zwischen beiden Zubereitungen wird nur durch Betätigung der Auslassöffnung (D) hergestellt.

Die Behältnisse (A) und (B) können innerhalb des Aerosol-Druckbehälters (C) neben, über oder untereinander angeordnet sein. Das Fassungsvermögen der Behältnisse (A) und (B) kann jeweils 10 cm³ bis 1000 cm³ betragen, und das Fassungsvermögen der Behältnisse (A) und (B) kann gleich oder unterschiedlich sein. Bevorzugt ist das Fassungsvermögen der Behältnisse (A) und (B) gleich. Der Aerosol-Druckbehälter (C) besitzt eine Auslassöffnung (D), die mit den beiden Behältnissen (A) und (B) in Verbindung steht. Während der Entnahme werden gleichzeitig die beiden Zubereitungen (A) und (B) über die gemeinsame Auslassöffnung entnommen und erst in oder direkt nach der Auslassöffnung (D) miteinander vermischt. Damit kommen die Zubereitungen (A) und (B) erst während oder direkt nach Durchtritt durch die Auslassöffnung (D) miteinander in Kontakt und bilden hierbei das anwendungsbereite Farbänderungsmittel.

Bei der Auslassöffnung (D) kann es sich zum Beispiel um ein Ventil handeln, und die Entnahme der Zubereitungen (A) und (B) über die gemeinsame Auslassöffnung (D) erfolgt durch Drücken des Ventils.

In einer weiteren bevorzugten Ausführungsform ist ein erfindungsgemäßes Produkt dadurch gekennzeichnet, dass
- das erste Behältnis (Behältnis A) und das zweite Behältnis (Behältnis B) sich zusammen im Inneren eines Aerosol-Druckbehälters (C) befinden,
- der Aerosol-Druckbehälter (C) eine Auslassöffnung (D) aufweist, die mit dem Behältnis (A) und mit dem Behältnis (B) in Verbindung steht und
- der Raum zwischen den Außenwandungen der beiden Behältnisse (A) und (B) und der Innenwandung des Aerosol-Druckbehälters (C) mit mindestens einem Treibgas aus der Gruppe Propan, Propen, n-Butan, iso-Butan, iso-Buten, n-Pentan, Penten, iso-Pentan, iso-Penten, Luft, Stickstoff, Argon, N₂O und/oder CO₂ enthält

In der vorgenannten bevorzugten Ausführungsform umfasst das erfindungsgemäße Produkt einen äußeren Druckgasbehälter. Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Besonders bevorzugte Druckgasbehälter sind Gefäße aus Metall (Aluminium, Weißblech, Zinn).

Wie zuvor beschrieben handelt es sich bei den beiden voneinander getrennten Behältnissen (A) und (B) besonders bevorzugt um zwei verformbare Beutel, vorzugsweise aus mit einem synthetischen Polymer (I) laminiertem Aluminium, welche jeweils mit der Auslassöffnung (D) in Verbindung stehen. Bei der Auslassöffnung (D) handelt es sich um eine Druckbehälter-Abgabevorrichtung. Beide Beutel befinden sich in einem dosenförmigen Druckbehälter, wobei der Druckbehälter zusammen mit der Druckbehälterabgabevorrichtung das Produkt nach außen druckdicht abschließt. Der Raum zwischen der Außenwandung der Beutel und der Innenwandung des Druckbehälters ist mit mindestens einem Treibgas befüllt. Entsprechende Spender sind auch beispielsweise aus US 2009/0108021 A1 bekannt.

Besonders gute erfindungsgemäße Effekte werden erzielt, wenn der Innendruck des Druckbehälters bei mindestens 1,8 bar, insbesondere mindestens 2,5 bar, beträgt.

Das Produkt umfasst weiterhin eine Abgabevorrichtung (entsprechend der Auslassöffnung D), welche zur Abgabe des Anwendungsmischung ein Ventil besitzen kann. In einer bevorzugten Ausführungsform der Erfindung weist das Ventil einen mit einem Lack oder einem polymeren Kunststoff beschichteten Ventilteller und ein ebensolches flexibles Element mit Rückstellcharakteristik auf, dass das Ventil nach Beenden der Betätigung in die Verschlussstellung (= Ruhelage des Ventils) zurückstellt. Entsprechende kosmetische Produkte, bei denen die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff beschichtet ist/sind, sind erfindungsgemäß ebenfalls bevorzugt.

In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element des Ventils mit Rückstellcharakteristik einstückig mit dem Ventilkegel ausgebildet sein und biegsame Beine aufweisen. Diese Feder kann aus Metall oder Kunststoff sein.

Alle erfindungsgemäß verwendeten Ventile weisen vorzugsweise einen innenlackierten Ventilteller auf, wobei Lackierung und Ventilmaterial miteinander kompatibel sind. Werden erfindungsgemäß Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Die eingesetzten Behälter, die z. B. aus Weißblech oder aus Aluminium sein können, wobei Aluminiumbehälter erfindungsgemäß bevorzugt sind, müssen angesichts der Korrosivität der erfindungsgemäß verwendeten Wasser in Öl Emulsionen ebenfalls innen lackiert oder beschichtet sein.

Da das erfindungsgemäße Produkt über einen Druckbehälter appliziert wird, enthalten die Spender zusätzlich mindestens ein Treibgas aus der Gruppe Propan, Propen, n-Butan, iso-Butan, iso-Buten, n-Pentan, Penten, iso-Pentan, iso-Penten, Luft, Stickstoff, Argon, N₂O und/oder CO₂.

Innerhalb dieser Gruppe sind die permanenten Gase Luft, Stickstoff, Argon, N₂O und/oder CO₂ bevorzugt, ganz besonders bevorzugt sind Stickstoff, Argon und/oder CO₂.

Weiterhin hat es sich als bevorzugt herausgestellt, wenn auch die Treibgase in bestimmten Drücken im Aerosol-Druckbehälter (C) enthalten sind. In einer bevorzugten Ausführungsform enthalten die erfindungsgemäßen Aerosol-Druckbehälter (C) ein oder mehrere weitere Treibgase daher mit einem Druck von 3 - 12 bar, bevorzugt von 4 bis 10 bar, und besonders bevorzugt von 5 bis 8 bar - jeweils bezogen auf den Druck der Treibgase zwischen den beutelförmigen Kammern (A) und (B) und dem Druckbehälter.

Vorzugsweise werden die einzelnen Produktzusammensetzungen im Zuge der Produktanwendungen zu einer Gesamtformulierung vermischt, um auf diesem Wege die vollständige Wirksamkeit der Gesamtformulierung auszuschöpfen sowie die Produktanwendung allgemein zu erleichtern. Zu diesem Zweck umfasst der Spender sinnvollerweise neben den Behältnissen (A), (B) einen Ausgabekopf, innerhalb dessen die Zubereitungen (A) und (B) von den Kammern zur Auslassöffnung (D) befördert werden. Dabei ist im Ausgabekopf eine geeignete Mischvorrichtung ausgebildet, welche stromaufwärts der Auslassöffnung (D) für die gewünschte Durchmischung der Zubereitungen (A) und (B) sorgt, bevor die durchmischte Gesamtformulierung, d. h. (A) + (B) über die Auslassöffnung (C) abgegeben wird. Beispielsweise aus der DE 3729491 A1 ist ein gattungsgemäßer Spender mit einer vergleichbaren Mischvorrichtung bekannt, wobei die dortige Mischvorrichtung jedoch nur eine sehr kurze Mischstrecke aufweist. Erfindungsgemäß ist eine derartige Mischvorrichtung unmittelbar in den Ausgabekopf baulich integriert oder aber alternativ als separates Bauteil innerhalb des Ausgabekopfes angeordnet. Als geeignete Mischvorrichtungen im Sinne der Erfindung sind beispielsweise Statikmischer oder vergleichbar wirkende Mischstrecken zu verstehen, die von den fließfähigen Zubereitungen (A) und (B) durchströmt werden und im Zuge dieser Durchströmung hinreichend vermischt werden. Dazu weist eine solche Mischstrecke üblicherweise geeignete Strömungseinbauten oder Strömungsstörer auf, die bei Umströmung aufgrund von erzeugten Turbulenzen eine Durchmischung einzelner Fluidkomponenten bewirken. Entscheidend für die Güte der Durchmischung einzelner Fluidkomponenten, hier der Zubereitungen (A) und (B), innerhalb der Mischvorrichtung ist u. a. die zielgerichtete Abstimmung der Länge der Mischstrecke sowie die Gestaltung der Strömungseinbauten auf die rheologischen Eigenschaften der Zubereitungen (A) und (B). Üblicherweise sind für hinreichende Mischergebnisse der erfindungsgemäßen Mischvorrichtung definierte Mindestlängen der Mischstrecke erforderlich. Daher ist die Mischstrecke der erfindungsgemäßen Mischvorrichtung bevorzugt derart gestaltet, dass zwar eine definierte Mindestlänge der Mischstrecke garantiert ist, ohne eine kompakte Gesamtkonstruktion der Mischvorrichtung und somit des Ausgabekopfes aufzugeben. So werden ausreichende Mischergebnisse gewährleistet und gleichzeitig gewünscht kompakte Außenabmessungen der Mischvorrichtung und des Ausgabekopfes sichergestellt. Dazu ist die Mischstrecke beispielsweise spiralförmig oder vergleichbar kompakt innerhalb des Ausgabekopfes ausgebildet.

Die Wahl der Volumina der einzelnen Behältnisse (A) und (B) richtet sich nach dem gewünschten Verhältnis der Volumina von Zubereitung (A) und Zubereitung (B). Bevorzugt sind die Volumina der Kammern (A) und (B) gleich.

Das Mengenverhältnis der Zubereitung (A) zur Menge der Zubereitung (B) liegt erfindungsgemäß bevorzugt in einem Bereich von 1:3 bis 3:1, ein Bereich von 1:1,5 bis 1,5:1 ist erfindungsgemäß bevorzugt, besonders bevorzugt ist ein Mengenverhältnis von 1:1.

Die erfindungsgemäßen Produkte können in Verfahren zur oxidativen Veränderung der Haarfarbe eingesetzt werden. Diese Verfahren zeichnen sich für den Verbraucher durch besonderen Anwendungskomfort aus, da die aufwändige und fehleranfällige Herstellung der Anwendungsmischungen durch den Verbraucher entfällt.

Bezüglich weiterer bevorzugter Ausführungsformen des erfindungsgemäßen Verfahrens gilt mutatis mutandis das zu dem erfindungsgemäßen Produkt Gesagte.

## Patentansprüche

1. Produkt zur oxidativen Farbveränderung keratinischer Fasern, umfassend mindestens zwei getrennt voneinander konfektionierte Zubereitungen (A) und (B), wobei
- die erste Zubereitung (A) in einem kosmetischen Träger
(A1) Wasserstoffperoxid und
(A2) mindestens einen Chelatbildner aus der Gruppe aus 1-Hydroxyethan-1,1-diphosphonsäure (HEDP), Ethylendiamintetramethylenphosphonsäure (EDTMP), Diethylentriaminpentamethylenphosphonsäure (DTPMP), Aminotrimethylenphosphonsäure (ATMP), N,N-Bis[2-[bis(carboxymethyl)amino]ethyl]glycin, Ethylendiamin-N,N'-dibernsteinsäure (EDDS), 2-Hydroxypropylendiamin-N,N'-dibernsteinsäure (HPDDS), Ethylendiamin-N,N'-diglutarsäure (EDDG), Ethylendiamin-N,N'-bis-(orthohydroxyphenyl)essigsäure (EDDHA) und/oder einem physiologisch verträglichen Salz hiervon enthält,
- die zweite Zubereitung (B) in einem kosmetischen Träger
(B1) mindestens ein Alkalisierungsmittel enthält,
- die Zubereitung (A) in einem ersten Behältnis (Behältnis A) konfektioniert ist, das mindestens zwei Schichten umfasst, wobei
- eine innere Schicht des Behältnisses A eine Schicht aus einem synthetischen Polymer (I) aus der Gruppe der Polyolefine umfasst und
- eine äußere Schicht des Behältnisses A eine Schicht aus Metall umfasst, und
- die Zubereitung (B) in einem zweiten Behältnis (Behältnis B) konfektioniert ist, das mindestens zwei Schichten umfasst, wobei
- eine innere Schicht des Behältnisses B eine Schicht aus einem synthetischen Polymer (I) aus der Gruppe der Polyolefine umfasst und
- eine äußere Schicht des Behältnisses B eine Schicht aus Metall umfasst,
- das erste Behältnis (Behältnis A) und das zweite Behältnis (Behältnis B) sich zusammen im Inneren eines Aerosol-Druckbehälters (C) befinden,
- der Aerosol-Druckbehälter (C) eine Auslassöffnung (D) aufweist, die mit dem Behältnis (A) und mit dem Behältnis (B) in Verbindung steht und
- der Raum zwischen den Außenwandungen der beiden Behältnisse (A) und (B) und der Innenwandung des Aerosol-Druckbehälters (C) mit mindestens einem Treibgas befüllt ist.

2. Produkt nach Anspruch 1, **dadurch gekennzeichnet, dass** die erste Zubereitung (A) - bezogen auf das Gesamtgewicht der Zubereitung (A) -
(A1) Wasserstoffperoxid in einer Menge von 0,5 bis 20,0 Gew.-%, bevorzugt 1,5 bis 17,0 Gew.-%, weiter bevorzugt von 1,5 bis 15,0 Gew.-% und besonders bevorzugt von 2,5 bis 12,0 Gew.-% enthält.

3. Produkt nach einem der Ansprüche 1 bis 2, **dadurch gekennzeichnet, dass** die erste Zubereitung (A) - bezogen auf das Gesamtgewicht der Zubereitung (A) - einen oder mehrere Chelatbildner (A2) in einer Gesamtmenge von 0,01 bis 5,5 Gew.-%, bevorzugt von 0,1 bis 4,5 Gew.-%, weiter bevorzugt von 0,3 bis 3,5 Gew.-% und besonders bevorzugt von 0,7 bis 2,5 Gew.-% enthält.

4. Produkt nach einem der Ansprüche 1 bis 3, **dadurch gekennzeichnet, dass** die erste Zubereitung (A) -
(A2) mindestens einen Chelatbildner aus der Gruppe aus 1-Hydroxyethan-1,1-diphosphonsäure (HEDP), Ethylendiamintetramethylenphosphonsäure (EDTMP), Diethylentriaminpentamethylenphosphonsäure (DTPMP), Aminotrimethylenphosphonsäure (ATMP), und/oder einem physiologisch verträglichen Salz hiervon enthält.

5. Produkt nach einem der Ansprüche 1 bis 4, **dadurch gekennzeichnet, dass** die erste Zubereitung (A) -
(A2) als Chelatbildner 1-Hydroxyethan-1,1-diphosphonsäure (HEDP und/oder ein physiologisch verträgliches Salz hiervon enthält.

6. Produkt nach einem der Ansprüche 1 bis 5, **dadurch gekennzeichnet, dass** das Gewichtsverhältnis aus der Gesamtmenge des in der Zubereitung (A) enthaltenen Wasserstoffperoxids (A1) zur Gesamtmenge der in der Zubereitung (A) enthaltenen Chelatbildner aus der Gruppe (A2), d.h. das Gewichtsverhältnis (A1)/(A2), bei einem Wert von 1,0 bis 30,0, bevorzugt von 2,0 bis 20,0, weiter bevorzugt von 3,0 bis 15,0 und besonders bevorzugt von 3,3 bis 13,5 liegt.

7. Produkt nach einem der Ansprüche 1 bis 6, **dadurch gekennzeichnet, dass** die erste Zubereitung (A) - bezogen auf das Gesamtgewicht der Zubereitung (A) - zusätzlich
(A3) 2,6-Dipicolinsäure, Benzoesäure und/oder Salicylsäure und/oder deren phyiologisch verträgliche Salze in einer Gesamtmenge von 0,05 bis 1,5 Gew.-%, bevorzugt 0,1 bis 1,0 Gew.-%, weiter bevorzugt von 0,2 bis 0,9 Gew.-% und besonders bevorzugt von 0,25 bis 0,7 Gew.-% enthält.

8. Produkt nach einem der Ansprüche 1 bis 7, **dadurch gekennzeichnet, dass**
- die erste Zubereitung (A) eine wässrige Zubereitung mit einem pH-Wert von 1,5 bis 5,0, bevorzugt von 2,0 bis 4,5, weiter bevorzugt von 2,2 bis 4,0 und besonders bevorzugt von 2,6 bis 3,5 ist und
- die zweite Zubereitung (B) eine wässrige Zubereitung mit einem pH-Wert von 7,5 bis 12,5, bevorzugt von 8,5 bis 11,5 und besonders bevorzugt von 8,9 bis 10,5 ist.

9. Produkt nach einem der Ansprüche 1 bis 8, **dadurch gekennzeichnet, dass**
- es sich bei dem ersten Behältnis (Behältnis A) um einen Beutel mit einer Gesamt-Schichtdicke von 2 bis 400 µm (Mikrometer), bevorzugt von 10 bis 250 µm (Mikrometer) und besonders bevorzugt von 50 bis 200 µm (Mikrometer) handelt und/oder
- es sich bei dem zweiten Behältnis (Behältnis B) um einen Beutel mit einer Gesamt-Schichtdicke von 2 bis 400 µm (Mikrometer) bevorzugt von 10 bis 250 µm (Mikrometer) und besonders bevorzugt von 50 bis 200 µm (Mikrometer) handelt.

10. Produkt nach einem der Ansprüche 1 bis 9, **dadurch gekennzeichnet, dass**
- die innere Schicht des Behältnisses A eine Schicht aus einem synthetischen Polymer (I) aus der Gruppe der Polyolefine aus Polyethylen, Polypropylen, einem Polyethylen/Polypropylen-Copolymer, Poly-1-buten, einem Polyethylen/Poly-1-buten-Copolymer, einem Polypropylen/Poly-1-buten-Copolymer und/oder einem Polyethylen/Polypropylen/Poly-1-buten-Copolymer umfasst, und/oder
- die innere Schicht des Behältnisses B eine Schicht aus einem synthetischen Polymer (I) aus der Gruppe der Polyolefine aus Polyethylen, Polypropylen, einem Polyethylen/Polypropylen-Copolymer, Poly-1-buten, einem Polyethylen/Poly-1-buten-Copolymer, einem Polypropylen/Poly-1-buten-Copolymer und/oder einem Polyethylen/Polypropylen/Poly-1-buten-Copolymer umfasst.

11. Produkt nach einem der Ansprüche 1 bis 10, **dadurch gekennzeichnet, dass**
- die innere Schicht des Behältnisses (A) eine Schicht aus einem synthetischen Polymer (I) mit einer Schichtdicke von 20 bis 200 µm (Mikrometer), bevorzugt von 40 bis 150 µm (Mikrometer) und besonders bevorzugt von 50 bis 80 µm (Mikrometer) umfasst, und/oder
- die innere Schicht des Behältnisses (B) eine Schicht aus einem synthetischen Polymer (I) mit einer Schichtdicke von 20 bis 200 µm (Mikrometer), bevorzugt von 40 bis 150 µm (Mikrometer) und besonders bevorzugt von 50 bis 80 µm (Mikrometer) umfasst.

12. Produkt nach einem der Ansprüche 1 bis 11, **dadurch gekennzeichnet, dass**
- die äußere Schicht des Behältnisses (A) eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) umfasst und/oder
- die äußere Schicht des Behältnisses (B) eine Schicht aus Aluminium mit einer Schichtdicke von 5 bis 50 µm (Mikrometer), bevorzugt von 7 bis 25 µm (Mikrometer) und besonders bevorzugt von 7 bis 12 µm (Mikrometer) umfasst.

13. Produkt nach einem der Ansprüche 1 bis 12, **dadurch gekennzeichnet, dass**
- die Behältnisse (A) und/oder (B) eine dritte Schicht aus einem synthetischen Polymer (II) umfassen, das strukturell von dem synthetischen Polymer (I) verschieden ist.

14. Produkt nach Anspruch 13, **dadurch gekennzeichnet, dass**
- die dritte Schicht des Behältnisses (A) eine Schicht aus einem synthetischen Polymer aus der Gruppe der Polyurethane, Polyester, Polyamide, Polyethylenterephthalate, Polybutylenterephthalate und der von Polymer (I) verschiedenen Polyolefine umfasst und/oder
- die dritte Schicht des Behältnisses (B) eine Schicht aus einem synthetischen Polymer aus der Gruppe der Polyurethane, Polyester, Polyamide, Polyethylenterephthalate, Polybutylenterephthalate und der von Polymer (I) verschiedenen Polyolefine umfasst.

15. Produkt nach einem der Ansprüche 1 bis 14, **dadurch gekennzeichnet, dass**
- das Behältnis (A) aus nicht mehr als 7, bevorzugt nicht mehr als 6, weiter bevorzugt nicht mehr als 5, noch weiter bevorzugt nicht mehr als 4 und ganz besonders bevorzugt nicht mehr als 3 verschiedenen Schichten besteht und/oder
- das Behältnis (B) aus nicht mehr als 7, bevorzugt nicht mehr als 6, weiter bevorzugt nicht mehr als 5, noch weiter bevorzugt nicht mehr als 4 und ganz besonders bevorzugt nicht mehr als 3 verschiedenen Schichten besteht.

## Claims

1. A product for oxidatively changing the color of keratin fibers, comprising at least two preparations (A) and (B) packaged separately from one another, wherein
- the first preparation (A) contains, in a cosmetic carrier,
(A1) hydrogen peroxide, and
(A2) at least one chelating agent from the group consisting of 1-hydroxyethane-1,1-diphosphonic acid (HEDP), ethylenediamine tetramethylene phosphonic acid (EDTMP), diethylenetriamine pentamethylene phosphonic acid (DTPMP), aminotrimethylene phosphonic acid (ATMP), N,N-bis[2-[bis(carboxymethyl)amino]ethyl]glycine, ethylenediamine-N,N'-disuccinic acid (EDDS), 2-hydroxypropylene diamine-N,N'-disuccinic acid (HPDDS), ethylenediamine-N,N'-diglutaric acid (EDDG), ethylenediamine-N,N'-bis-(orthohydroxyphenyl) acetic acid (EDDHA) and/or a physiologically acceptable salt thereof,
- the second preparation (B) contains, in a cosmetic carrier,
(B1) at least one alkalizing agent,
- preparation (A) is packaged in a first container (container A) which comprises at least two layers, wherein
- an inner layer of container A comprises a layer made of a synthetic polymer (I) from the group of polyolefins, and
- an outer layer of container A comprises a layer made of metal, and
- preparation (B) is packaged in a second container (container B) which comprises at least two layers, wherein
- an inner layer of container B comprises a layer made of a synthetic polymer (I) from the group of polyolefins, and
- an outer layer of container B comprises a layer made of metal,
- the first container (container A) and the second container (container B) are located together inside a pressurized aerosol container (C),
- the pressurized aerosol container (C) has an outlet opening (D) which is connected to container (A) and to container (B), and
- the space between the outer walls of the two containers (A) and (B) and the inner wall of the pressurized aerosol container (C) is filled with at least one propellant.

2. The product according to claim 1, **characterized in that** the first preparation (A) contains, based on the total weight of preparation (A),
(A1) hydrogen peroxide in an amount of 0.5 to 20.0 wt.%, preferably 1.5 to 17.0 wt.%, more preferably of 1.5 to 15.0 wt.% and particularly preferably of 2.5 to 12.0 wt.%.

3. The product according to one of claims 1 to 2, **characterized in that** the first preparation (A) contains, based on the total weight of preparation (A), one or more chelating agents (A2) in a total amount of 0.01 to 5.5 wt.%, preferably of 0.1 to 4.5 wt.%, more preferably of 0.3 to 3.5 wt.%, and particularly preferably of 0.7 to 2.5 wt.%.

4. The product according to one of claims 1 to 3, **characterized in that** the first preparation (A) contains
(A2) at least one chelating agent from the group consisting of 1-hydroxyethane-1,1-diphosphonic acid (HEDP), ethylenediamine tetramethylene phosphonic acid (EDTMP), diethylenetriamine pentamethylene phosphonic acid (DTPMP), aminotrimethylene phosphonic acid (ATMP), and/or a physiologically acceptable salt thereof.

5. The product according to one of claims 1 to 4, **characterized in that** preparation (A) contains
(A2) 1-hydroxyethane-1,1-diphosphonic acid (HEDP) and/or a physiologically acceptable salt thereof as a chelating agent.

6. The product according to one of claims 1 to 5, **characterized in that** the weight ratio of the total amount of the hydrogen peroxide (A1) contained in preparation (A) to the total amount of the chelating agents contained in preparation (A) from the group (A2), i.e., the weight ratio (A1)/(A2), is at a value from 1.0 to 30.0, preferably from 2.0 to 20.0, more preferably from 3.0 to 15.0 and particularly preferably from 3.3 to 13.5.

7. The product according to one of claims 1 to 6, **characterized in that** the first preparation (A) additionally contains, based on the total weight of preparation (A),
(A3) 2,6-dipicolinic acid, benzoic acid and/or salicylic acid and/or the physiologically acceptable salts thereof in a total amount of from 0.05 to 1.5 wt.%, preferably from 0.1 to 1.0 wt.%, more preferably from 0.2 to 0.9 wt.%, and particularly preferably from 0.25 to 0.7 wt.%.

8. The product according to one of claims 1 to 7, **characterized in that**
- the first preparation (A) is an aqueous preparation having a pH of 1.5 to 5.0, preferably of 2.0 to 4.5, more preferably of 2.2 to 4.0, and particularly preferably of 2.6 to 3.5, and
- the second preparation (B) is an aqueous preparation having a pH of 7.5 to 12.5, preferably of 8.5 to 11.5 and particularly preferably of 8.9 to 10.5.

9. The product according to one of claims 1 to 8, **characterized in that**
- the first container (container A) is a bag having a total layer thickness of 2 to 400 µm (micrometers), preferably of 10 to 250 µm (micrometers) and particularly preferably of 50 to 200 µm (micrometers), and/or
- the second container (container B) is a bag having a total layer thickness of 2 to 400 µm (micrometers), preferably of 10 to 250 µm (micrometers) and particularly preferably of 50 to 200 µm (micrometers).

10. The product according to one of claims 1 to 9, **characterized in that**
- the inner layer of container A comprises a layer made of a synthetic polymer (I) from the group of polyolefins consisting of polyethylene, polypropylene, a polyethylene/polypropylene copolymer, poly-1-butene, a polyethylene/poly-1-butene copolymer, a polypropylene/poly-1-butene copolymer and/or a polyethylene/polypropylene/poly-1-butene copolymer, and/or
- the inner layer of container B comprises a layer made of a synthetic polymer (I) from the group of polyolefins consisting of polyethylene, polypropylene, a polyethylene/polypropylene copolymer, poly-1-butene, a polyethylene/poly-1-butene copolymer, a polypropylene/poly-1-butene copolymer and/or a polyethylene/polypropylene/poly-1-butene copolymer.

11. The product according to one of claims 1 to 10, **characterized in that**
- the inner layer of container (A) comprises a layer made of a synthetic polymer (I) having a layer thickness of 20 to 200 µm (micrometers), preferably of 40 to 150 µm (micrometers) and particularly preferably of 50 to 80 µm (micrometers), and/or
- the inner layer of container (B) comprises a layer made of a synthetic polymer (I) having a layer thickness of 20 to 200 µm (micrometers), preferably of 40 to 150 µm (micrometers) and particularly preferably of 50 to 80 µm (micrometers).

12. The product according to one of claims 1 to 11, **characterized in that**
- the outer layer of container (A) comprises a layer made of aluminum having a layer thickness of 5 to 50 µm (micrometers), preferably of 7 to 25 µm (micrometers) and particularly preferably of 7 to 12 µm (micrometers), and/or
- the outer layer of container (B) comprises a layer made of aluminum having a layer thickness of 5 to 50 µm (micrometers), preferably of 7 to 25 µm (micrometers) and particularly preferably of 7 to 12 µm (micrometers).

13. The product according to one of claims 1 to 12, **characterized in that**
- containers (A) and/or (B) comprise a third layer made of a synthetic polymer (II) which is structurally different from the synthetic polymer (I).

14. The product according to claim 13, **characterized in that**
- the third layer of container (A) comprises a layer made of a synthetic polymer from the group consisting of polyurethanes, polyesters, polyamides, polyethylene terephthalates, polybutylene terephthalates, and polyolefins different from polymer (I), and/or
- the third layer of container (B) comprises a layer made of a synthetic polymer from the group of polyurethanes, polyesters, polyamides, polyethylene terephthalates, polybutylene terephthalates, and polyolefins different from polymer (I).

15. The product according to one of claims 1 to 14, **characterized in that**
- container (A) consists of not more than 7, preferably not more than 6, more preferably not more than 5, even more preferably not more than 4 and very particularly preferably not more than 3 different layers, and/or
- container (B) consists of not more than 7, preferably not more than 6, more preferably not more than 5, even more preferably not more than 4 and very particularly preferably not more than 3 different layers.

## Revendications

1. Produit permettant la coloration par oxydation de fibres kératiniques, comprenant au moins deux préparations (A) et (B) conditionnées séparément,
- la première préparation (A) contenant, dans un support cosmétique,
(A1) du peroxyde d'hydrogène, et
(A2) au moins un agent chélatant du groupe constitué par l'acide 1-hydroxyéthane-1,1-diphosphonique (HEDP), l'acide éthylènediaminetétraméthylènephosphonique (EDTMP), l'acide diéthylènetriaminepentaméthylènephosphonique (DTPMP), l'acide aminotriméthylènephosphonique (ATMP), la N,N-bis[2-[bis(carboxyméthyl)amino]éthyl]glycine, l'acide éthylènediamine-N,N'-disuccinique (EDDS), l'acide 2-hydroxypropylènediamine-N,N'-disuccinique (HPDDS), l'acide éthylènediamine-N,N'-diglutarique (EDDG), l'acide éthylènediamine-N,N'-bis-(orthohydroxyphényl)acétique (EDDHA) et/ou un sel physiologiquement acceptable de ceux-ci,
- la seconde préparation (B) contenant, dans un support cosmétique,
(B1) au moins un agent alcalinisant,
- la préparation (A) étant conditionnée dans un premier récipient (récipient A) qui comprend au moins deux couches,
- une couche intérieure du récipient A comprenant une couche constituée d'un polymère synthétique (I) du groupe des polyoléfines, et
- une couche extérieure du récipient A comprenant une couche en métal, et
- la préparation (B) étant conditionnée dans un second récipient (récipient B) qui comprend au moins deux couches,
- une couche intérieure du récipient B comprenant une couche constituée d'un polymère synthétique (I) du groupe des polyoléfines, et
- une couche extérieure du récipient B comprenant une couche en métal,
- le premier récipient (récipient A) et le second récipient (récipient B) étant présents ensemble à l'intérieur d'un contenant aérosol sous pression (C),
- le contenant aérosol sous pression (C) présentant une ouverture de sortie (D) qui est reliée au récipient (A) et au récipient (B), et
- l'espace entre les parois externes des deux récipients (A) et (B) et la paroi interne du contenant aérosol sous pression (C) étant rempli d'au moins un gaz propulseur.

2. Produit selon la revendication 1, **caractérisé en ce que** la première préparation (A) contient - par rapport au poids total de la préparation (A) -
(A1) le peroxyde d'hydrogène en une quantité de 0,5 à 20,0 % en poids, de préférence de 1,5 à 17,0 % en poids, de manière davantage préférée de 1,5 à 15,0 % en poids et de manière particulièrement préférée de 2,5 à 12,0 % en poids.

3. Produit selon l'une des revendications 1 à 2, **caractérisé en ce que** la première préparation (A) contient - par rapport au poids total de la préparation (A) - un ou plusieurs agents chélatants (A2) en une quantité totale de 0,01 à 5,5 % en poids, de préférence de 0,1 à 4,5 % en poids, de manière davantage préférée de 0,3 à 3,5 % en poids et de manière particulièrement préférée de 0,7 à 2,5 % en poids.

4. Produit selon l'une des revendications 1 à 3, **caractérisé en ce que** la première préparation (A) contient
(A2) au moins un agent chélatant du groupe constitué par l'acide 1-hydroxyéthane-1,1-diphosphonique (HEDP), l'acide éthylènediaminetétraméthylènephosphonique (EDTMP), l'acide diéthylènetriaminepentaméthylènephosphonique (DTPMP), l'acide aminotriméthylènephosphonique (ATMP) et/ou un sel physiologiquement acceptable de ceux-ci.

5. Produit selon l'une des revendications 1 à 4, **caractérisé en ce que** la première préparation (A) contient,
(A2) comme agents chélatants, de l'acide 1-hydroxyéthane-1,1-diphosphonique (HEDP) et/ou un sel physiologiquement acceptable de celui-ci.

6. Produit selon l'une des revendications 1 à 5, **caractérisé en ce que** le rapport pondéral de la quantité totale du peroxyde d'hydrogène (A1) contenu dans la préparation (A) à la quantité totale des agents chélatants contenus dans la préparation (A) issus du groupe (A2), c'est-à-dire le rapport pondéral (A1)/(A2), est une valeur de 1,0 à 30,0, de préférence de 2,0 à 20,0, de manière davantage préférée de 3,0 à 15,0 et de manière particulièrement préférée de 3,3 à 13,5.

7. Produit selon l'une des revendications 1 à 6, **caractérisé en ce que** la première préparation (A) contient également - par rapport au poids total de la préparation (A) -
(A3) de l'acide 2,6-dipicolinique, de l'acide benzoïque et/ou de l'acide salicylique
et/ou un sel physiologiquement acceptable de ceux-ci en une quantité de 0,05 à 1,5 % en poids, de préférence de 0,1 à 1,0 % en poids, de manière davantage préférée de 0,2 à 0,9 % en poids et de manière particulièrement préférée de 0,25 à 0,7 % en poids.

8. Produit selon l'une des revendications 1 à 7, **caractérisé en ce que**
- la première préparation (A) est une préparation aqueuse ayant un pH de 1,5 à 5,0, de préférence de 2,0 à 4,5, de manière davantage préférée de 2,2 à 4,0 et de manière particulièrement préférée de 2,6 à 3,5, et
- la seconde préparation (B) est une préparation aqueuse ayant un pH de 7,5 à 12,5, de préférence de 8,5 à 11,5 et de manière particulièrement préférée de 8,9 à 10,5.

9. Produit selon l'une des revendications 1 à 8, **caractérisé en ce que**
- le premier récipient (récipient A) est un sachet d'une épaisseur totale de couche de 2 à 400 µm (micromètres), de préférence de 10 à 250 µm (micromètres) et de manière particulièrement préférée de 50 à 200 µm (micromètres), et/ou
- le second récipient (récipient B) est un sachet d'une épaisseur totale de couche de 2 à 400 µm (micromètres), de préférence de 10 à 250 µm (micromètres) et de manière particulièrement préférée de 50 à 200 µm (micromètres).

10. Produit selon l'une des revendications 1 à 9, **caractérisé en ce que**
- la couche intérieure du récipient (A) est une couche constituée d'un polymère synthétique (I) du groupe des polyoléfines, de polyéthylène, de polypropylène, d'un copolymère de polyéthylène/polypropylène, de poly-1-butène, d'un copolymère de polyéthylène/poly-1-butène, d'un copolymère de polypropylène/poly-1-butène et/ou d'un copolymère de polyéthylène/polypropylène/poly-1-butène, et/ou
- la couche intérieure du récipient (B) est une couche constituée d'un polymère synthétique (I) du groupe des polyoléfines, de polyéthylène, de polypropylène, d'un copolymère de polyéthylène/polypropylène, de poly-1-butène, d'un copolymère de polyéthylène/poly-1-butène, d'un copolymère de polypropylène/poly-1-butène et/ou d'un copolymère de polyéthylène/polypropylène/poly-1-butène.

11. Produit selon l'une des revendications 1 à 10, **caractérisé en ce que**
- la couche intérieure du récipient (A) comprend une couche constituée d'un polymère synthétique (I) ayant une épaisseur de couche de 20 à 200 µm (micromètres), de préférence de 40 à 150 µm (micromètres) et de manière particulièrement préférée de 50 à 80 µm (micromètres), et/ou
- la couche intérieure du récipient (B) comprend une couche constituée d'un polymère synthétique (I) ayant une épaisseur de couche de 20 à 200 µm (micromètres), de préférence de 40 à 150 µm (micromètres) et de manière particulièrement préférée de 50 à 80 µm (micromètres).

12. Produit selon l'une des revendications 1 à 11, **caractérisé en ce que**
- la couche extérieure du récipient (A) comprend une couche constituée d'aluminium ayant une épaisseur de couche de 5 à 50 µm (micromètres), de préférence de 7 à 25 µm (micromètres) et de manière particulièrement préférée de 7 à 12 µm (micromètres), et/ou
- la couche extérieure du récipient (B) comprend une couche constituée d'aluminium ayant une épaisseur de couche de 5 à 50 µm (micromètres), de préférence de 7 à 25 µm (micromètres) et de manière particulièrement préférée de 7 à 12 µm (micromètres).

13. Produit selon l'une des revendications 1 à 12, **caractérisé en ce que**
- les récipients (A) et/ou (B) comprennent une troisième couche constituée d'un polymère synthétique (II) qui est structurellement différent du polymère synthétique (I).

14. Produit selon la revendication 13, **caractérisé en ce que**
- la troisième couche du récipient (A) comprend une couche constituée d'un polymère synthétique du groupe des polyuréthanes, polyesters, polyamides, polyéthylène téréphtalates, polybutylène téréphtalates et des polyoléfines différentes du polymère (I), et/ou
- la troisième couche du récipient (B) comprend une couche constituée d'un polymère synthétique du groupe des polyuréthanes, polyesters, polyamides, polyéthylène téréphtalates, polybutylène téréphtalates et des polyoléfines différentes du polymère (I).

15. Produit selon l'une des revendications 1 à 14, **caractérisé en ce que**
- le récipient (A) est constitué d'au plus 7, de préférence d'au plus 6, de manière davantage préférée d'au plus 5, encore plus préférablement d'au plus 4 et de manière tout particulièrement préférée d'au plus 3 couches différentes, et/ou
- le récipient (B) est constitué d'au plus 7, de préférence d'au plus 6, de manière davantage préférée d'au plus 5, encore plus préférablement d'au plus 4 et de manière tout particulièrement préférée d'au plus 3 couches différentes.
